(19) Europäisches Patentamt
European Patent Office
Office européen des brevets

(11) EP 2 190 409 B1

(12) **EUROPEAN PATENT SPECIFICATION**

(45) Date of publication and mention of the grant of the patent:
**24.10.2018 Bulletin 2018/43**

(21) Application number: **08788693.3**

(22) Date of filing: **18.08.2008**

(51) Int Cl.:
***A61K 9/00*** *(2006.01)* ***A61K 9/16*** *(2006.01)*
***A61K 31/00*** *(2006.01)*

(86) International application number:
**PCT/GB2008/050722**

(87) International publication number:
**WO 2009/022190 (19.02.2009 Gazette 2009/08)**

(54) **DELIVERY OF DRUG COMBINATIONS**

VERABREICHUNG VON ARZNEIMITTELKOMBINATIONEN

ADMINISTRATION DE COMBINAISONS DE MÉDICAMENTS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **16.08.2007 GB 0716003**
**20.11.2007 EP 07121158**

(43) Date of publication of application:
**02.06.2010 Bulletin 2010/22**

(73) Proprietor: **Biocompatibles UK Limited**
**Farnham**
**Surrey GU9 8QL (GB)**

(72) Inventors:
- **LEWIS, Andrew Lennard**
  **Farnham, Surrey GU9 8QL (GB)**
- **FORSTER, Richard, Edward, John**
  **Singapore 129206 (SG)**
- **GONZALEZ-FAJARDO, Maria Victoria**
  **28015 Madrid (ES)**
- **TANG, Yiqing**
  **Farnham, Surrey GU9 8QL (GB)**
- **LLOYD, Andrew William**
  **Brighton, Sussex BN2 4GJ (GB)**
- **PHILLIPS, Gary, James**
  **Brighton, Sussex BN2 4GJ (GB)**

(74) Representative: **BTG plc Intellectual Property Group**
**5 Fleet Place**
**London EC4M 7RD (GB)**

(56) References cited:
**WO-A-2004/071495 WO-A-2006/027567**

- **PASUT ET AL: "Polymer-drug conjugation, recent achievements and general strategies" PROGRESS IN POLYMER SCIENCE, PERGAMON PRESS, OXFORD, GB, vol. 32, no. 8-9, 7 August 2007 (2007-08-07), pages 933-961, XP022188828 ISSN: 0079-6700**

EP 2 190 409 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to drug delivery articles, usually microspheres, formed of a polymer matrix and at least two drugs, the drugs being releasable from the polymer matrix. The drugs are for treatment of cancer, especially solid tumours.

Combination therapy has been the basis for most success stories in cancer treatment, which is understandable when components of a combination have a favourable pharmacological interaction (same target, but different large-organ toxicities). Combination therapy with multiple drugs is a clinically common procedure in tumour treatment. The most common therapeutic regimens for ovarian, prostate or uterine cancer are based in combination of two antitumor drugs. This type of therapy could offer the advantage of increasing the tumoricidal efficacy and reducing the side effects caused by the high-dose of a single drug. Irinotecan is indicated in combination with 5-FU for the treatment of patients with advanced colorectal cancer and it has been used with success in combination with other agents such as epirubicin, mitomycin or capecitabine to treat breast cancer (Becerra 2004, Oncology, 18, 46-48), ovarian cancer (Nishino et al. 2005, Gynecol Oncol, 97, 893-897) or gastric cancer (Baek et al. 2006, Br J Cancer, 25, 312-320) respectively.

A more recent foundation for the success of some cancer treatments involves a mechanistic basis, under which a combination of drugs is used to affect different molecular targets to circumvent resistance. A barrier to major advancements in combination therapy, however, has been a lack of understanding about the intersection of critical signalling pathways. Synergy might be induced through the effect of drugs on the same as well as parallel pathways. Because the number of drug combinations is limitless, a strategy for determining the most promising combinations and prioritizing their evaluation is crucial.

Mathematical modelling and computer simulations are used to calculate the expected effect of different drug combinations in an effort to better understand how chemotherapeutic combinations might be used clinically. In vivo, favourable synergistic drug combinations may result in increased efficacy, decreased dosage, reduced toxicity, and minimized development of resistance. The Median Effect Equation (Chou J. Biol. Chem. 1977, 252(18), 6438-6442) and Combination Index Equation (Chou-Talalay T.I.P.S. Nov. 1983 450-454) are two such treatments in which the isobologram curves generated represent additive, synergistic, or antagonistic interactions caused by different drug combinations. The concept behind and applications of surface response models encompasses the joint effect of two active agents which can be viewed as a three-dimensional response surface. While these models for assessing joint action are the most comprehensive, they are also highly complex and difficult to create. Indeed, it is recognised that the true link between empirical synergy and the underlying biochemical, molecular, and physiological mechanisms is very difficult to discover.

A fluorescence-based, high-throughput system, called digital image microscopy-based cytotoxicity assay (DIMSCAN), exists for in vitro testing of cytostatic or cytotoxic drug combinations. There are five principles for this type of testing: (a) the assay system should have a wide dynamic range, ideally 3 to 4 logs of cell kill; (b) the cell line panel should employ multiple cell lines, including drug-resistant lines; (c) major mechanisms of resistance should be identified and used to structure the cell line panel; (d) exposure to drugs should be at clinically achievable levels and schedules; and (e) because hypoxia may antagonize drug action, it is essential to test under hypoxic conditions. The best approach for selecting drug combinations for evaluation is the one based on molecular targets; studies should first be performed in vitro and then confirmed with in vivo models before clinically testing the drug combination. Drug antagonism is also frequently used to exclude drug combinations.

There are known synergistic interactions between novel signal transduction modulators and established cytotoxic agents, cell cycle inhibitors, and differentiation inducers. While the first type of drug combination involves toxic concentrations of the cytotoxic agent to produce modest potentiation in vitro, the second and third types induce a high degree of synergism at concentrations of both agents that are nontoxic (ineffective) when given individually. Drug combinations between cell cycle inhibitors or these agents and differentiation inducers have also been shown to produce significant synergistic activity in vitro. The number of signal transduction modulator combinations associated with synergistic interactions of cell death is virtually limitless. Whether these combinations are therapeutically superior to those between conventional cytotoxic agents or those between conventional and novel agents and whether they represent a new paradigm for combination chemotherapy remains to be determined.

Celator Technologies, Inc., in Canada, have reported on commercial solutions for bringing effective drug combinations to the clinic. Celator is working on the identification of critical ratios at which drug combinations act synergistically to kill tumour cells, with the objective of fixing those synergistic ratios in a liposome-based delivery vehicle (CombiPlex) designed to target tumors following i.v. injection. Here, whilst the *in vitro* synergistic activity depends on specific drug ratios, the in vivo activity depends on maintaining these synergistic ratios. The drugs are incorporated jointly into the liposomes. Their systems have been described in patent publications, such as WO2003028696, WO2004087115, WO2004087105, WO2004093795, WO2006055903, and in Tardi, P. G. et al, (2007) Biochim. Biophys. Acta 1768, 678-587.

Microspheres offer a particularly versatile platform for delivery of drug combinations. Sharma et al have investigated microspheres for inhalation of drug combinations to treat tuberculosis (Sharma R, et al, 2001, Pharm Research, 18, 1405-1410). Drug therapy of tuberculosis (TB) requires long-term oral administration of multiple drugs for curing as well as preventing and/or combating multi-drug resistance. Persistent, high blood levels of antitubercular drugs resulting from prolonged oral administration of anti-TB drugs may be neither necessary nor sufficient to kill mycobacteria residing in macrophages (Mϕ). Inhalable biodegradable microparticles containing two of the first-line anti-TB drugs, isoniazid (H), and rifampicin (R), were prepared and tested for (i) phagocytosis by mouse Mϕ (ii) administration as a dry powder inhalation to rats, and (iii) targeting alveolar Mϕ with high drug doses when administered to rats. Results showed that such microspheres with multiple drugs offer promise for dose and dose-frequency reduction, toxicity alleviation and targeting of Mϕ-resident persistent mycobacteria.

In another study (Gupte A & Ciftci K, 2004, Int. J. Pharm,. 276(1-2):93-106.), the combination (Paclitaxel + 5-FU microspheres) was compared with a single drug chemotherapy (Paclitaxel and 5-FU microspheres) against metastatic breast cancer cell line (MDA-MB 435 S). The physicochemical characteristics of the microspheres (i.e. encapsulation efficiency, particle size distribution, in vitro release, thermal characteristics) were studied. The results demonstrated that the encapsulation efficiency of Paclitaxel was high (90%) when the drug was encapsulated in poly(lactic-co-glycolic acid) (PLGA) microparticles with or without 5-fluorouracil (5-FU). However, the encapsulation efficiency of 5-FU was low (19%) and increased to 30% when the drug was encapsulated with Paclitaxel. The mean particle size of microspheres was 2.5 μm and were spherical in shape. The in vitro release of both 5-FU and Paclitaxel from the microspheres was relatively fast initially followed by a slower and more controlled release. The cytotoxic activity of Paclitaxel microspheres was far greater compared to either the microspheres containing 5-FU + Paclitaxel or 5-FU alone. Overall results demonstrated that incorporation of Paclitaxel or 5-FU in microspheres enhances the cytotoxicity in more controlled manner compared to that of free drugs and also that careful consideration should be made when combining drugs acting in different phases of cell cycle.

Others have investigated drug combinations in microspheres to target restenosis (Chandy et al, 2001, Development of Poly(Lactic Acid)/Chitosan Co-Matrix Microspheres: Controlled Release of Taxol-Heparin for Preventing Restenosis, Drug delivery, 8, 77-86). Smooth muscle cell proliferation plays a major role in the genesis of restenosis after angioplasty or vascular injury. Controlled release of appropriate drugs alone and in combinations is one approach for treating coronary obstructions, balloon angioplasty, restenosis associated with thrombosis, and calcification. Chady et al, demonstrated the possibility of encapsulating taxol-loaded polylactic acid (PLA) microspheres within heparin-chitosan spheres to develop a prolonged release co-matrix form. The in vitro release profile of taxol and heparin from this co-matrix system was monitored in phosphate buffered saline pH 7.4, using an ultraviolet spectrophotometer. The amount of taxol/heparin release was initially much higher, followed by a constant slow release profile for a prolonged period. The initial burst release of taxol (15.8%) and heparin (32.7%) from the co-matrix was modified with polyethylene glycol coatings (13.5% and 25.4%, respectively, for 24 hr). From scanning electron microscopy studies, it appears that these drugs diffuse out slowly to the dissolution medium through the micropores of the co-matrix. However, the surface micropores were modified with polyethylene glycol (PEG) coatings for a constant slow release profile. They concluded that this PEG-coated PLA/chitosan co-matrix may target drug combinations having synergistic effects for prolonged periods to treat restenosis.

Cardiovascular stents coated with a combination of drugs have been described for reducing restenosis rate. Restenosis is a hyper proliferative disease. For instance EP-A-551162 discloses a combination of rapamycin and mycophenolic acid, an anti-proliferative agent, but does not give details of how a stent is to be impregnated with the actives. In EP-A-0568130 rapamycin and heparin are administered to prevent smooth muscle hyperplasia, e.g. by delivery from an impregnated stent. No details are given of impregnation methods.

WO-A-2003022807 describes administration of a rapamycin analogue from a stent coated with a polymer. The drug is loaded by dipping the polymer coated stent into a solution of the drug and drying. In addition a further drug such as an anti-miotic, anti-proliferative or anti-inflammatory agent may be administered from the stent. Polymers include among many others polyvinyl alcohol, hyaluronic acid and copolymers of phosphorylcholine methacrylate (MPC), non-ionic comonomers and crosslinking monomers, and these last mentioned copolymers are exemplified in *in vitro* tests, with a rapamycin analogue alone. In WO2004/022124 a rapamycin analogue and a dexamethasone are jointly loaded into a crosslinked MPC copolymer with non-ionic comonomer on a stent. The polymer used in these specifications has a water-swellability of about 50%.

5-Fluorouracil (5-FU) in combination with leucovorin (LV) is nowadays the standard treatment in colon cancer. Lamprecht et al (Eur J Pharm and Biopharm, 2005, 59, 367-371) used Eudragit P-4135F or Eudragit RS100 separately to prepare microspheres by an oil/oil emulsification process trapping 5-FU and LV simultaneously. Scanning electron microscopy permitted a structural analysis, process parameters were analyzed and drug loading and release profiles were recorded. Particle size varied between 123 (RS100) and 146 μm (P-4135F). Generally, higher encapsulation rates were found with RS100 (5-FU, 60.3±9.7%; LV, 81.4±8.6%) compared to P-4135F (5-FU, 48.3±2.0%; LV, 55.4±2.7%). These Eudragit polymers are acrylic copolymers including quaternary ammonium group containing alkyl methacrylate monomers. They are not crosslinked. Microparticles made from Eudragit RS100 released the incorporated drug combination within 8 h. P-4135F was found to maintain the undesired 5-FU release at pH 6.8 lower than 25% within 4 h while at pH 7.4, a nearly immediate release (within 15 min) was observed. Although the release was similar at pH 7.4, at pH 6.8 LV showed a distinct initial drug loss of about 60% and a complete release within 2 h. SEM analyses revealed a substantial presence of LV crystals on the particle surface provoking a distinct burst effect of LV. These observations were concluded to be related to the high lipophilicity of P-4135F provoking a separation between P-4135F and LV during the preparation process.

Liu et al. (J. Pharm. Pharmacol., 55, 1063-1073, 2003) and in WO9850018 describe the use of biodegradable sulfopropyl dextran microspheres for intratumoural injection. In the method microspheres containing doxorubicin are co-administered with microspheres containing verapamil. Following intratumoural injection in mice of doxorubicin-loaded microspheres, alone or in combination with verapamil-loaded microspheres, the tumour diameter was measured serially as an indication of therapeutic effect, while the weight, appearance, and behaviour of the mice were monitored as an indication of general toxicity. Intratumoural injections of doxorubicin-loaded microspheres were tolerated much better than systemic administration of equivalent drug concentrations. There was a modest (up to 34%) delay of tumour growth compared with groups receiving no treatment or blank microspheres. Co-injection of verapamil microspheres with doxorubicin microspheres produced a moderate increase in toxicity but no further delay in tumour growth. Controlled-release microsphere formulations of anticancer agents administered intratumourally were described as an efficient way to deliver high drug doses to the tumour with little systemic toxicity. The sulpho-propylated dextran microspheres used had been supplied as ion-exchange media (Sephadex SP25). They are biodegradable. They are swellable in distilled water to give a volume ratio of microspheres in the swollen state to dry state of about 12. The microspheres have an ion-exchange capacity of about 2.5 meq/g at pH 7.

Liu et al. in (2000) J. Pharm. Sci: 8-9(6) 807-817 have also described ways of modifying the surface of the sulpho-propylated microspheres with hydrophobic moieties or with cationic polymers. Cationic polymer modification reduces the swellability ratio and ion-exchange capacity. The surface conjugation of hydrophobic groups affects the rate of swelling in water but does not reduce the extent of swelling at equilibrium verapamil loaded at a more rapid rate into the polymer treated microspheres.

Doxorubicin eluting beads have been shown to be effective in the treatment of HCC by chemoembolisation (Varela et al., 2007, J Hepatology, 46, 474-81). Microspheres for delivering doxorubicin to solid tumours by chemoembolisation are described in WO2004071495. The microspheres are formed of a matrix of water-swellable anionically charged crosslinked polyvinyl alcohol and have diameters when swollen in water in the range 40 to 1500 μm.

Ibuprofen eluting beads have demonstrated anti-inflammatory effects post embolisation in animal models (Borovac T, et al, 2006, J Control Release, 115, 266-74). Microspheres for use in the treatment of uterine fibroids loaded with ibuprofen are described in WO2004073688. Improved loading methods are described in WO2007085615.

Ibuprofen is an non-steroidal anti-inflammatory drug (NSAID), which acts by inhibition of the enzymes cyclooxygenase I and II. Many authors have studied the use of NSAIDs for treatment and prevention of cancer risk (Sawaoka et al. 1998, J Clin Gastroenterol, 27, 47-52; Shen et al. 1998, Cancer Research, 58, 362-366). COX-2 selective NSAIDs have also demonstrated a reduction in cancer cell invasion and liver metastases (Yamauchi et al. 2003, Anticancer Res, 23, 245-249; Yao et al. 2004, Br J Cancer, 90, 712-719) and inhibition of COX-2 resulted in reduced tumour size and increased survival (Cui et al. 2005, Clin Cancer Res, 11, 8213-8221; Murata et al. 2005, Dig Dis Sci, 50, 70-75). Ibuprofen has been investigated in the treatment and prevention of a number of tumours. In WO2006013376 we describe the use of microspheres loaded with various COX inhibitors for embolisation showing elution rates and localisation which lead to an expectation that an effect would be shown on tumourigenesis.

In WO2007090897, we describe methods of loading water-swellable polymers, such as microspheres, with drugs having a very low solubility, but which are soluble in an organic solvent, in which a solvent based solution of the drug is contacted with substantially dry polymer matrix, so that swelling of the matrix in the drug solution takes place, followed by precipitation of the drug in the matrix of the polymer by contacting with a precipitant, usually water. The method is suitable for loading paclitaxel, dexamethasone or rapamycin. The polymer matrix is a crosslinked polyvinyl alcohol based material, usually having pendent anionic groups, such as sulphonate groups.

In WO2006027567 we describe microspheres, for chemoembolisation of solid tumours comprising a water-swellable polymer which is anionically charged at pH 7 and, electrostatically associated with the polymer in releasable form, a cationically charged camptothecin compound, such as irinotecan. We have shown that chemoembolisation using these beads for treatment of liver metastases from colorectal cancer leads to a reduction in tumour volume.

In WO 2004/071495 we describe a composition for chemoembotheapy of solid tumours comprising particles of a water-insoluble, water-swellable synthetic anionic polymer and, absorbed therein an anthracycline. Preferably the polymer is a poly (vinyl alcohol) based polymer and the drug is doxorubicin.

According to a first aspect of the present invention there is provided a composition comprising a population of microspheres each of which has a polymer matrix and, jointly incorporated into the matrix, a first and a second pharmaceutically active compound, wherein the polymer is a water-insoluble water-swellable polymer which is anionically charged, the first active is cationically charged and the second active is electrostatically neutral, and one of the actives

is a cytotoxic compound and the other active has activity complementary to the cytotoxic compound in tumour treatment.

In this aspect of the invention, the first cationically charged active may be the cytotoxic compound. Alternatively the second active may be the cytotoxic compound, while the first cationic active has activity complementary to the cytotoxic compound in tumour treatment. Ionically charged actives tend to be water-soluble at a relatively high level, for instance at a level of at least 50 g/l, preferably at least 100 g/l. The second active, which is electrostatically neutral, may be less water-soluble, for instance having a low solubility in water, less than 10 g/l, at room temperature. We have found that for the combination of cationic and nonionic drugs, there is little or no interference with the loading or release rate of each active by the other. This result is surprising and allows the ready determination of suitable dosages and ratios of composition which are optimised for efficacy. The composition will contain particles of a single type which avoids problems of separation causing dosage uncertainties.

The second active has an activity which is complementary to the cytotoxic compound in tumour treatment. The compound may itself be efficacious in treatment of tumours, for instance be capable of preventing growth or reducing the size of a tumour, for instance when delivered locally, or systemically. The active may alternatively be a compound which sensitises the tumour to activity by the first active. In general the combination of compounds may be identified as one which has a combination index based on the Chou-Talalay index (1983, *op. cit*), of less than 0.90, more preferably less than 0.5, for instance less than 0.3, even as low as 0.1 or below.

Values for the combination index are determined, conveniently, using the software package Calcusyn (Biosoft), which is a dose effect analysis programme utilising the general equation for dose effect relationships derived by Chou:

$$f_a/f_u = (D/D_m)^m \qquad \text{Eq. 1.}$$

where D: the dose of drug

$D_m$: the median-effect dose signifying the potency. It is determined from the x-intercept of the median-effect plot.

$f_a$: the fraction affected by the dose

$f_u$: the fraction unaffected, $fu=1-f_a$

m: an exponent signifying the sigmoidicity (shape) of the dose effect curve. It is determined by the slope of the median effect plot.

The alternative forms of the median-effect equation are:

$$f_a = 1/[1+ (D_m/D)^m] \qquad \text{Eq. 2.}$$

$$D = D_m[f_a/(1-f_a)]^{1/m} \qquad \text{Eq. 3.}$$

From Eq. 2, if $D_m$ and m are known, the effect ($f_a$) can be determined for any dose (D).

From Eq. 3, if Dm and m are known, the dose (D) or ($D_x$) can be determined for any effect ($f_a$). Thus, $D_m$ and m parameters representing the potency and shape, respectively, determine the entire dose-effect curve.

The median-effect plot is a plot of x = log (D) vs y = log ($f_a/f_u$) introduced by Chou in 1976 (Chou J. Th. Biol. 59, 253-276). It is based on the logarithmic form of Chou's median effect equation [Eq. 1]:

$$\log (f_a/f_u) = m \log (D) - m \log (D_m) \qquad \text{Eq. 4}$$

Eq. 4 has the form of a straight line, y = mx + b.

Thus, the slope yields m value and the x-intercept yields log ($D_m$) value, and thus, the $D_m$ value. The $D_m$ can also be determined by: $D_m = 10^{-(\text{y-intercept})/m)}$.

Note that $f_a/f_u = f_a/(1-f_a) = (f_u)^{-1}-1 = [(f_a)^{-1}-1]^{-1}$. The goodness of fit for the data to the median-effect equation is represented by the linear correlation coefficient r of the median-effect plot. Usually, the experimental data from enzyme or receptor systems have r > 0.96, from tissue culture r > 0.90; and from animal systems, r > 0.85.

The combination index (CI) equation is based on the multiple drug-effect equation of Chou-Talalay derived from enzyme kinetic models. An equation determines only the additive effect rather than synergism or antagonism. However, we define synergism as a more than expected additive effect, and antagonism as a less than expected additive effect. Chou and Talalay in 1983 (*op. cit*) proposed the designation of CI = 1 as the additive effect, thus from the multiple drug effect equation of two drugs, we obtain:

Classical: for mutually exclusive drugs that have the same or similar modes of action:

$$CI = (D)_1/(D_x)_1 + (D)_2/(D_x)_2 \qquad \text{Eq. 5}$$

Conservative: for mutually non-exclusive drugs that have totally independent modes of action:

$$CI = (D)_1/(D_x)_1 + (D)_2/(D_x)_2 + (D)_1(D)_2/(D_x)_1(D_x)_2 \qquad \text{Eq. 6}$$

Eq. 5 or Eq. 6 dictates that drug 1, $(D)_1$, and drug 2, (D)2, (in the numerators) in combination inhibit x% in the actual experiment. Thus, the experimentally observed x% inhibition may not be around number but most frequently has a decimal fraction. $(D_x)_1$ and $(D_x)_2$ (in the denominators) of Eq. 5 or 6 are the doses of drug 1 and drug 2 alone, respectively, inhibiting x%. $D_x$ can be readily calculated from Eq. 3.

It is worth noting that each drug alone should have a dose-effect relationship. Both potency ($D_m$) and shape (m) parameters are essential for determining synergism or antagonism. If one of the two drugs has no effect by itself, then synergism/antagonism cannot be determined. Instead, potentiation (augmentation, enhancement) or inhibition (suppression) can be determined.

Calcusyn requires the dose-effect relationship for each drug and then for combinations. Although the experimental data from the combined study is preferably collected using a constant ratio, combination indices may nevertheless be determined from non-constant ratios of the two components.

The combination index may be determined for any *in vitro or in vivo* test method. In the present invention, it is most convenient for the combination index to be determined *in vitro* using a cell culture based test, for instance on a cell line of cells representing a useful *in vitro* test for determining *in vivo* efficacy. For instance the test may determine the cytotoxicity of the individual drugs and then of the combination, and these data used to calculate the combination index. In the test, the drugs may be contacted with the cells in solution, or in some other form. The effect which is to be measured is cytotoxicity, such as reduction in 50% viable cell count compared to control after a given period such as 24 or 48 hours. It is not necessary for the drugs to be incorporated into a polymer matrix, for instance in the form of microspheres, to carry out the test. However it is preferred that the combination index is determined to be less than 1 when the drugs are delivered from a polymer matrix, for instance in the form of microspheres as defined in the present claims. In this case, the combination of drugs tested may involve separate particles, each loaded with one of the drugs or each particle may be jointly loaded with both the drugs. A suitable test is described in Examples 6 and 7 below. Where separate particles are each loaded with one of the drugs it should be checked that, for the joint-loaded particles the elution rate of each drug is not significantly affected by the other if it is to be assumed that the determined CI value will apply to the joint-loaded product. In this aspect of the invention the combination of pharmaceutically active compounds is one which has a CI of less than 1.0 in an *in vitro* cytotoxicity test on a tumour cell line at a ratio within a multiple of 5 (either way) of the ratio in the composition. Where the composition is for treatment of a hepatocellular carcinoma the cell line used to determine the CI is preferably a hepatocellular carcinoma cell line. Where the composition is for treating pancreatic carcinoma, the cell line is preferably a pancreatic carcinoma cell line. The CI should preferably be the defined low value when the active compounds are tested at a ratio within a multiple of 2 either way of the ratio in the composition.

A cytotoxic agent is one that is directly toxic to cells, preventing their reproduction or growth. Suitable cytotoxic agents are, for instance, anthracycline compounds such as doxorubicin and other compounds disclosed in WO04071495, camptothecin derivatives as described in WO2006027567, taxanes, platinum-based neoplastic anti-metabolites such as 5-FU, mercaptopurine, capecitabine other cytotoxic antibiotics such as actinomycin D and vinca alkaloids, including vinblastine, vincristine, vindesine and vinorelbine. Examples of cytarabine, gemcitabine, cyclophosphamide, fludaribine, camptothecin compounds such as topotecan and irinotecan, clorambucil, busulfan, mitoxantrone, retinoids, anagrelide etc.

The active which provides complementary activity may be a cytostatic compound. Cytostatic compounds may be defined as compounds that inhibit or suppress cellular growth or reproduction. The cytotoxic and cytostatic effects of drugs may be determined by the person skilled in the art, using suitable tests, such as based on cell lines.

Suitable cytostatic compounds are rapamycin and analogs.

Suitable cationically charged compounds are: doxorubicin and other anthracycline compounds having an amine group as disclosed in WO04071495 or mitoxantrone, cationic camptothecin derivatives, such as irinotecan, as described in WO2006027567, or topotecan, or verapamil.

One class of cytotoxic or cytostatic compounds which may be used comprises rapamycin and rapamycin analogues, which target mTOR. Such compounds include sirolimus, temsirolimus, everolimus, biolimus, ABT-578 and AP23573. Any of the compounds encompassed within the scope of rapamycin analogues described in WO-A-2003022807, may be used as the rapamycin analogue.

Where the cytotoxic active is rapamycin or a rapamycin derivative/analogue, the other active is in one preferred embodiment, doxorubicin or another anthracycline compound having an amine group as described in WO04071495.

The combination of anticancer drug rapamycin and doxorubicin has been demonstrated a complete remission of a mouse model of B-cell lymphoma through the reverse of chemoresistance in lymphomas by rapamycin sensitisation, and induction of apoptosis by doxorubicin (Wendel,H-G, et al, 2004, Nature 428, 332-337). In the treatment of leukemias, rapamycin has shown the enhancement of doxorubicin-induced apoptosis (Avellino,R. et al, 2005, Blood 106(4), 1400-1406). Combinations of rapamycin or an analogue with an anthracycline compound having at least one amine group show a low combination index, and are highly effective in the present invention.

In another embodiment of this aspect of the present invention the cytotoxic compound is rapamycin or an analogue and the cationic active is a camptothecin derivative having a cationic substituent as disclosed in WO2006027567 or in EP-A-0321122. Preferably the active is as defined in claim 6. Preferably A is COO. Preferably AR is substituted in the 10 position.

According to the first aspect of the present invention, the two drugs are jointly loaded onto the same microspheres. Where the cytotoxic compound is rapamycin or an analogue, the active having complementary activity may alternatively be selected from cisplatin, or other platinum based anti-cancer drug, gemcitabine, tamoxifen or another anti-estrogen, interferon alpha, epithelial growth factor receptor inhibitor, vascular endothelial growth factor inhibitor, inhibitors of both VEGFR and EGFR, and Glivec. The further drug may alternatively be paclitaxel or an analogue, or other microtubule inhibitor, a topoisomerase inhibitor.

Combinations of rapamycin with other anticancer agents is also known, and the known combination disclosed below may be used in this aspect of the invention. Cisplatin, gemcitabine, tamoxifen, interferon alpha, EGFR inhibitor, VEGFR/EGFR inhibitor and Gleevec have all been combined with rapamycin analogues in preclinical or clinical studies, and these combinations may be used in the further aspect of the invention.

Interactions between rapamycin and chemotherapeutic agents used as first- and second-line agents against breast cancer have been investigated. In vitro, synergistic interactions were observed in combinations with paclitaxel, carboplatin, and vinorelbine. Additive effects were observed in combinations with doxorubicin and gemcitabine. Rapamycin dramatically enhanced paclitaxel- and carboplatin-induced apoptosis. This effect was sequence dependent and mediated at least partly through caspase activation. Furthermore, rapamycin enhanced chemosensitivity to paclitaxel and carboplatin in HER2/neu-overexpressing cells, suggesting a potential approach to these poorly behaving tumours. In vivo, rapamycin combined with paclitaxel resulted in a significant reduction in tumour volume compared with either agent alone in rapamycin-sensitive tumours. (Mondesire WH, et al., 2004, Clinical Cancer Research, 10, 7031-7042). The combination of a rapamycin or analogue with a taxane may be used in the further aspect of the invention.

RAD001 dramatically enhances cisplatin-induced apoptosis in wt p53, but not mutant p53 tumour cells. RAD001 sensitises cells to cisplatin by inhibiting p53-induced p21 expression. These findings support the molecular rationale for combining DNA damaging agents such as the platinum cytotoxic with RAD001, showing that a general major anabolic process may enhance the efficacy of an established drug protocol in the treatment of patients with solid tumours (Beuvink I et al 2005, Cell, 120:747-759). The combination of rapamycin or analogues with a platinum anti-neoplastic compound may be used in the further aspects of the invention.

CCI-779 and tamoxifen or another anti-estrogen may be useful if administered in combination. The PI3K/AKY/mTOR pathway may play an important role in the development of tamoxifen resistance in breast cancer. Activation of the estrogen receptor can drive the PI3K pathway. Cross talk between erb-1 and ER has been shown to activate the pathway, which has been associated with estrogen-independent transcriptional activity, and breast cancer cell lines with activated AKT are resistant to the growth inhibitory effects of tamoxifen. The combination of rapamycin or an analogue with an anti-estrogen may be used in this further aspect of the invention.

There is an ongoing phase III trial comparing orally administered temsirolimus (CCI-779) plus letrozole (an aromatase inhibitor) versus letrozole alone as first-line treatment among approximately 1200 postmenopausal women with advanced breast cancer. The combination of rapamycin or an analogue with an aromatase inhibitor may be used in this further aspect of the invention.

CCI-779 and interferon alpha have been used in conjunction in treatment of renal cell carcinoma (RCC). A large phase 3 trial is ongoing. The combination of rapamycin or an analogue and alpha interferon may be used in this further aspect of the invention.

Rapamycin with epidermal growth factor receptor (EGFR) inhibitor EKI-785 resulted in synergistic growth inhibition. Similar results were achieved with RAD-001 and VEGFR/EGFR inhibitor AEE788. Rapamycin with Glivec resulted in synergistic growth inhibition of CML cell lines. The combination of rapamycin or an analogue with a growth factor receptor or with Glivec may be used in this further aspect of the invention.

Other combinations used in the further aspect of the invention include cytotoxic agents with P-glycoprotein inhibitors. Approximately 50% of cancer patients receive chemotherapy, and as many as 75% of these patients experience intrinsic or acquired resistance to a broad spectrum of chemotherapeutic agents. This phenomenon, termed multidrug resistance (MDR), is the most common cause of chemotherapy failure. It is well established that the majority of tumours

develop MDR through over-expression of the drug efflux pump P-glycoprotein (P-gp). Inhibition of P-gp is acknowledged as a viable means of reversing MDR; however, existing P-gp inhibitors so far have demonstrated limited clinical success due to limitations in potency and specificity. Verapamil, a calcium channel blocker, is commonly used for the treatment of supraventricular arrhythmias, coronary heart disease, and arterial hypertension. Furthermore, verapamil is a potent inhibitor of P-glycoprotein-mediated transport, and has been proven to modify MDR with cancer chemotherapy in in vitro experiments (Toffoli G, et al. 1995, Biochem Pharmacol, 50, 1245-55; Pauli-Magnus C, et al. 2000, J Pharmacol Exp Ther, 293, 376-82). Local delivery of doxorubicin and verapamil from jointly loaded drug eluting beads may therefore offer a synergistic benefits, allowing high local doses of cytotoxic agent and P-gp inhibition to overcome any MDR effects. Thus the combination of a cytotoxic antibiotic and a P-gp inhibitor may be used in the further aspect of this invention.

Other combinations include topoisomerase inhibitors, such as tecans with other actives having low Combination Index.

Other combinations as described by Celator in any of WO2003028696, WO2004087115, WO2004087105, WO2004093795, WO2006055903, and in Tardi, P. G. et al., (2007) Biochim. Biophys. Acta 1768, 678-587, the contents of which are incorporated herein by reference, may be used in the further aspect of the invention.

Taxanes and anti-metabolites may be used in combination, such as paclitaxel and 5-FU. 5FU and leucovorin may be used.

Where the actives include a camptothecin derivative, it is particularly effective to use a derivative having cationic substituents, such as compounds of the general formula I

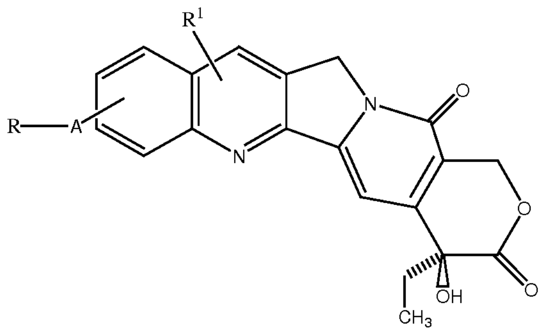

I

in which $R^1$ is selected from H, halogen, hydroxyl and lower ($C_{1-6}$) alkyl, optionally substituted by a hydroxyl, amine, alkoxy, halogen, acyl and acyloxy groups;

A is C(O)O or $CH_{2;}$ and

R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form a optionally substituted heterocyclic ring which may be interrupted by -O-, -S- or >$NR^4$ in which $R^4$ is a hydrogen atom, a substituted or unsubstituted $C_{1-4}$ alkyl group or a substituted or unsubstituted phenyl group;

and wherein the grouping -A-R is bonded to a carbon atom located in any of the 9, 10 or 11 positions in the A ring of the camptothecin compound and $R^1$ is substituted in the A or B ring, including salts thereof.

In one embodiment the camptothecin derivatives have the formula IA

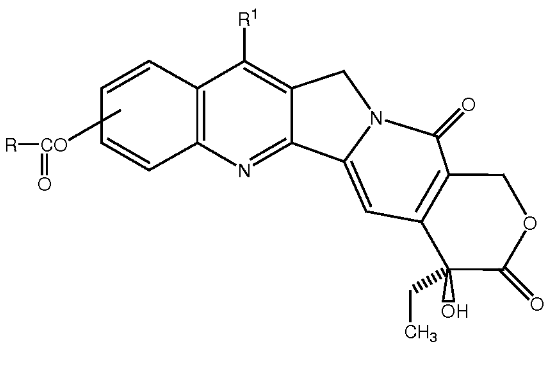

IA

in which $R^1$ is H, lower ($C_{1-6}$) alkyl, optionally substituted by a hydroxyl, amine, alkoxy, halogen, acyl or acyloxy group or halogen; and

R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form a optionally substituted heterocyclic ring which may be interrupted by -O-, -S- or >$NR^4$ in which $R^4$ is a hydrogen atom, a substituted or unsubstituted $C_{1-4}$ alkyl group or a substituted or unsubstituted phenyl group;
and wherein the grouping -O-CO-R is bonded to a carbon atom located in any of the 9, 10 or 11 positions in the A ring of the camptothecin compound, including salts thereof.

It is preferred for the grouping -O-CO-R to be joined at the 10 position.
$R^1$ is preferably $C_{1-4}$ alkyl, most preferably ethyl, and m is preferably 1.
A halogen atom R is, for instance, F, Cl, Br or I, preferably F or Cl. $R^1$ to $R^4$ may be methyl, ethyl, propyl, isopropyl, in-butyl, isobutyl and t-butyl, preferably methyl.
Substituents in R and $R^1$ are preferably selected from halogen atoms, hydroxy, $C_{1-4}$ alkoxy, phenoxy, $COOR^6$, $SO_3R^6$ and $PO_3(R^6)_2$, aryl,

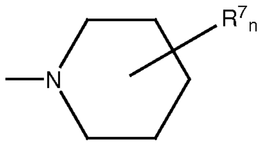
,

$NR^8R^9$ and $CONR^8R^9$, $QAOR^5$, $QANR^8R^9$ and $QAQR^5$ in which $R^5$ is $C_{1-4}$ alkyl or aryl; $R^6$ is hydrogen, halogen $C_{1-4}$ alkyl or $C_{1-4}$ alkoxy; $R^7$ is hydrogen, halogen or $C_{1-4}$ alkyl; $R^8$ and $R^9$ are the same or different and each is H, or $C_{1-4}$ alkyl or $R^8$ and $R^9$ together represent $C_{3-6}$ alkanediyl;
Q is OCO, or -COO- and A is $C_{2-4}$ alkanediyl.
Preferably R is $NR^3R^3$ where $R^2$ and $R^3$ together with the nitrogen atom form a 5 or 6 membered ring, preferably a saturated ring, with optional substituents. A substituent is preferably $-NR^8R^9$. In such a substituent $R^8$ and $R^9$ preferably together are $C_{4-5}$ alkanediyl. Such groups are basic and tend to be cationically charged at pH7. Most preferably R is

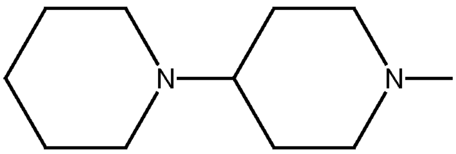

Another family of suitable compounds has the general formula II

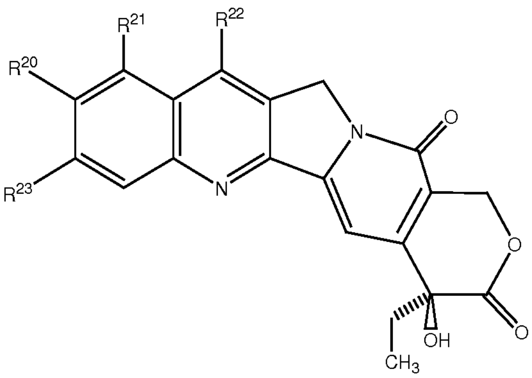
II

in which $R^{20}$ and $R^{23}$ are each hydroxy or hydrogen or together are $CH_2OCH_2$;
one of $R^{21}$ and $R^{22}$ is H and the other is $CH_2NR^{24}R^{25}$ where $R^{23}$ and $R^{24}$ are the same or different and each represents a hydrogen atom, a substituted or unsubstituted $C_{1-4}$ alkyl group or a substituted or unsubstituted carbocyclic or heterocyclic group, or $R^{23}$ and $R^{24}$ together with the nitrogen atom to which they are attached form a optionally substituted heterocyclic ring which may be interrupted by -O-, -S- or >$NR^4$ in which $R^4$ is a hydrogen atom, a substituted or unsubstituted $C_{1-4}$ alkyl group or a substituted or unsubstituted phenyl group; including salts and quaternary derivatives thereof. One example of a suitable compound of this claim is topotecan, in which $R^{20}$ is hydroxyl, $R^{22}$ and $R^{23}$ are hydrogen, $R^{21}$ is $CH_2NR^{24}R^{25}$ and $R^{24}$ and $R^{25}$ are both methyl.

The ratio of the first active to the second active is preferably in the range 100:1 to 1:100, preferably in the range 5:1 to 1:5. Suitable ratios may be determined by a person skilled in the art, for instance by using information available from combination index determinations. Generally the ratios should be selected so as to optimise the synergistic effect.

Preferably the ratio of equivalents of anion in the polymer to cation in the first active, should be in the range 2:1 to 1:1, preferably around 1:1. By selecting these ratios optimum loading may be achieved, while minimising the first effect upon initial administration, and providing optimised extended release.

The second active used in the invention may alternatively be a COX-inhibitor. COX-inhibitors, for instance NSAIDs, could act as anti-inflammatory and analgesic agents, targeting inflammation and pain associated with the chemoembolisation procedure. Furthermore, up to 50% of the cells in a tumour can be inflammatory cells. Also there have been numerous studies linking inflammation with angiogenesis and tumour progression. Accordingly a combination of a cytotoxic agent such as doxorubicin, with a COX-inhibitor may have highly desirable effects. As indicated above, ibuprofen has even been proposed as having useful effects on reducing cancer cell invasion and liver metastases.

The polymer is a water-insoluble material. Although it may be biodegradable, so that drug may be released substantially by erosion of polymer matrix to release drug from the surface, preferably the polymer is substantially biostable (i.e. non-biodegradable).

The polymer is water-swellable. Water-swellable polymer useful in the invention preferably has an equilibrium water content, when swollen in water at 37°C, measured by gravimetric analysis, in the range of 40 to 99 wt%, preferably 75 to 95%.

Generally the polymer is covalently crosslinked, although it may be appropriate for the polymer to be ionically crosslinked, at least in part, or hydrophobically crosslinked. In some embodiments it may be suitable to use polymers which are derived from natural sources, such as albumin, alginate, gelatin, starch, chitosan or collagen, all of which have been used as embolic agents. In other embodiments the polymer is substantially free of naturally occurring polymer or derivatives. It is preferably formed by polymerising ethylenically unsaturated monomers in the presence of di- or higher-functional crosslinking monomers. The ethylenically unsaturated monomers may include an ionic (including zwitterionic) monomer.

Copolymers of hydroxyethyl methacrylate, acrylic acid and cross-linking monomer, such as ethylene glycol dimethacrylate or methylene bisacrylamide, as used for etafilcon A based contact lenses may be used. Copolymers of N-acryloyl-2-amino-2-hydroxymethyl-propane-1,3-diol and N,N-bisacrylamide may also be used.

Other suitable synthetic polymers are cross-linked styrenic polymers e.g. with ionic substituents, of the type used as separation media or as ion-exchange media.

Another type of synthetic polymer which may be used to form the water-swellable water-insoluble matrix is polyvinyl alcohol crosslinked using aldehyde-type crosslinking agents such as glutaraldehyde. For such products, the polyvinyl alcohol (PVA) may be rendered ionic by providing pendant ionic groups by reacting a functional ionic group containing compound with the hydroxyl groups. Examples of suitable functional groups for reaction with the hydroxyl groups are acylating agents, such as carboxylic acids or derivatives thereof, or other acidic groups which may form esters.

The invention is of particular value where the polymer matrix is formed from a polyvinyl alcohol macromer, having more than one ethylenically unsaturated pendant group per molecule, by radical polymerisation of the ethylenic groups. Preferably the PVA macromer is copolymerised with ethylenically unsaturated monomers for instance including a nonionic and/or ionic monomer including anionic monomer.

The PVA macromer may be formed, for instance, by providing PVA polymer of a suitable molecular weight, such as in the range 1000 to 500,000 D, preferably 10,000 to 100,000 D, with pendant vinylic or acrylic groups. Pendant acrylic groups may be provided, for instance, by reacting acrylic or methacrylic acid with PVA to form ester linkages through some of the hydroxyl groups. Other methods for attaching vinylic groups capable of polymerisation onto polyvinyl alcohol are described in, for instance, US 4,978,713 and, preferably, US 5,508,317 and 5,583,163. Thus the preferred macromer comprises a backbone of polyvinyl alcohol to which is linked, via a cyclic acetal linkage, an (alk)acrylaminoalkyl moiety. Example 1 describes the synthesis of an example of such a macromer known by the approved name nelfilcon B. Preferably the PVA macromers have about 2 to 20 pendant ethylenic groups per molecule, for instance 5 to 10.

Where PVA macromers are copolymerised with ethylenically unsaturated monomers including an ionic monomer, the ionic monomer preferably has the general formula II

$$Y^1BQ^1 \qquad \text{II}$$

in which $Y^1$ is selected from

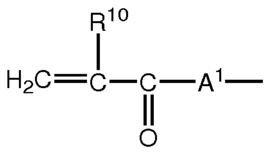

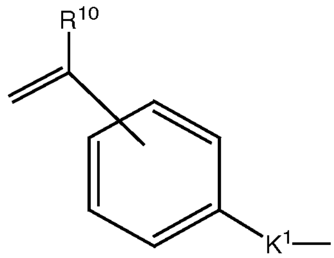

$CH_2=C(R^{10})-CH_2-O-$, $CH_2=C(R^{10})-CH_2\ OC(O)-$, $CH_2=C(R^{10})OC(O)-$, $CH_2=C(R^{10})-O-$, $CH_2=C(R^{10})CH_2OC(O)N(R^{11})-$, $R^{12}OOCCR^{10}=CR^{10}C(O)-O-$, $R^{10}CH=CHC(O)O-$, $R^{10}CH=C(COOR^{12})CH_2-C(O)-O-$,

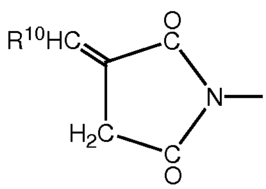

and

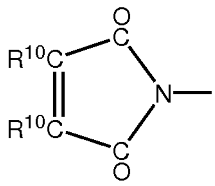

wherein:

- $R^{10}$ is hydrogen or a $C_1$-$C_4$ alkyl group;
- $R^{11}$ is hydrogen or a $C_1$-$C_4$ alkyl group;
- $R^{12}$ is hydrogen or a $C_{1-4}$ alkyl group or $BQ^1$ where B and $Q^1$ are as defined below;
- $A^1$ is -O- or $-NR^{11}-$;
- $K^1$ is a group $-(CH_2)_rOC(O)-$, $-(CH_2)_rC(O)O-$, $-(CH_2)_rOC(O)O-$, $-(CH_2)_rNR^{13}-$, $-(CH_2)_rNR^{13}C(O)-$, $-(CH_2)_rC(O)NR^{13}-$, $-(CH_2)_rNR^{13}C(O)O-$, $-(CH_2)_rOC(O)NR^{13}-$, $-(CH_2)_rNR^{13}C(O)NR^{13}-$ (in which the groups $R^{13}$ are the same or different), $-(CH_2)_rO-$, $-(CH_2)_rSO_3-$, or, optionally in combination with B, a valence bond and r is from 1 to 12 and $R^{13}$ is hydrogen or a $C_1$-$C_4$ alkyl group;
- B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if $Q^1$ or $Y^1$ contains a terminal carbon atom bonded to B a valence bond; and
- $Q^1$ is an anionic group.

An anionic group $Q^1$ may be, for instance, a carboxylate, carbonate, sulphonate, sulphate, nitrate, phosphonate or phosphate group. The monomer may be polymerised as the free acid or in salt form. Preferably the $pK_a$ of the conjugate acid is less than 5.

A suitable cationic group $Q^1$ is preferably a group $N^+R^{14}_3$, $P^+R^{15}_3$ or $S^+R^{15}_2$ in which the groups $R^{14}$ are the same or different and are each hydrogen, $C_{1-4}$-alkyl or aryl (preferably phenyl) or two of the groups $R^{14}$ together with the heteroatom to which they are attached from a saturated or unsaturated heterocyclic ring containing from 5 to 7 atoms the groups $R^{15}$ are each $OR^{14}$ or $R^{14}$. Preferably the cationic group is permanently cationic, that is each $R^{14}$ is other than hydrogen. Preferably a cationic group Q is $N^+R^{14}_3$ in which each $R^{14}$ is $C_{1-4}$-alkyl, preferably methyl.

A zwitterionic group $Q^1$ may have an overall charge, for instance by having a divalent centre of anionic charge and monovalent centre of cationic charge or vice-versa or by having two centres of cationic charge and one centre of anionic charge or vice-versa. Preferably, however, the zwitterion has no overall charge and most preferably has a centre of monovalent cationic charge and a centre of monovalent anionic charge.

Examples of zwitterionic groups which may be used as $Q^1$ in the present invention are disclosed in WO-A-0029481.

Where the ethylenically unsaturated monomer includes zwitterionic monomer, for instance, this may increase the hydrophilicity, lubricity, biocompatibility and/or haemocompatibility of the particles. Suitable zwitterionic monomers are described in our earlier publications WO-A-9207885, WO-A-9416748, WO-A-9416749 and WO-A-9520407. Preferably a zwitterionic monomer is 2-methacryloyloxy-2'-trimethylammonium ethyl phosphate inner salt (MPC).

In the monomer of general formula I preferably $Y^1$ is a group $CH^2=CR^{10}COA-$ in which $R^{10}$ is H or methyl, preferably methyl, and in which $A^1$ is preferably NH. B is preferably an alkanediyl group of 1 to 12, preferably 2 to 6 carbon atoms. Such monomers are acrylic monomers.

There may be included in the ethylenically unsaturated monomer diluent monomer, for instance non-ionic

monomer. Such a monomer may be useful to control the $pK_a$ of the acid groups, to control the hydrophilicity or hydrophobicity of the product, to provide hydrophobic regions in the polymer, or merely to act as inert diluent. Examples of non-ionic diluent monomer are, for instance, alkyl (alk) acrylates and (alk) acrylamides, especially such compounds having alkyl groups with 1 to 12 carbon atoms, hydroxy, and di-hydroxy-substituted alkyl(alk) acrylates and -(alk) acrylamides, vinyl lactams, styrene and other aromatic monomers.

In the polymer matrix, the level of anion is preferably in the range 0.1 to 10 meq $g^{-1}$, preferably at least 1.0 meq $g^{-1}$. Preferred anions are derived from strong acids, such as sulphates, sulphonates, phosphates and phosphonates.

Where PVA macromer is copolymerised with other ethylenically unsaturated monomers, the weight ratio of PVA macromer to other monomer is preferably in the range of 50:1 to 1:5, more preferably in the range 20:1 to 1:2. In the ethylenically unsaturated monomer the anionic monomer is preferably present in an amount in the range 10 to 100 mole%, preferably at least 25 mole%.

The crosslinked polymer is formed as such in microsphere form, for instance by polymerising in droplets of monomer in a dispersed phase in a continuous immiscible carrier. Examples of suitable water-in-oil polymerisations to produce particles having the desired size, when swollen, are known. For instance US 4,224,427 describes processes for forming uniform spherical beads (microspheres) of up to 5 mm in diameter, by dispersing water-soluble monomers into a continuous solvent phase, in a presence of suspending agents. Stabilisers and surfactants may be present to provide control over the size of the dispersed phase particles. After polymerisation, the crosslinked microspheres are recovered by known means, and washed and optionally sterilised. Preferably the particles e.g. microspheres, are swollen in an aqueous liquid, and classified according to their size.

Methods for loading the two actives into a microsphere are selected according to the nature of the actives and the loading levels required.. Where they are loaded simultaneously, this is suitable from a solution containing both the actives in combination with one another. Sequential loading methods may involve contact of one of the actives in an aqueous solution with polymer at a level or for a time such that ion-exchange within the polymer matrix takes place to an extent such that a proportion of the anionic groups of the polymer remain unassociated with cationic active. In a second step, optionally after removal of non-absorbed first active, a solution of the second active, usually in water, is contacted with the partially loaded microspheres for a period of time allowing ion-exchange to take place and the second drug to become loaded into the polymer.

Where one of the actives is a compound having low water-solubility, a different technique is used. For instance it may be possible to load the actives sequentially, for instance by contacting the microspheres with solutions of each drug in suitable solvents, enabling the microspheres to swell and absorb the active into the polymer matrix. In one embodiment, a low water-solubility active is loaded into the polymer first. In another embodiment, the cationically charged active is loaded first.

It may be possible to select a solvent or solvent system in which both a cationically charged active and a neutral active, which has low water-solubility, are soluble, the solvent being one which acts to swell the polymer. For instance, where the actives are both soluble in selected alcohol, the alcohol may be used to dissolve the actives together, and to swell the polymer matrix which is swellable in the selected alcohol.

Preferably the loading process involves the steps:

contacting microspheres which comprise a matrix of a water-insoluble water-swellable anionically charged crosslinked polymer in substantially dry form with a solution of a drug having a water-solubility less than 10 g/l at room temperature in a first organic solvent, whereby a solution of drug in solvent becomes impregnated into the microspheres;
separating drug solution which has not impregnated the microspheres;
contacting the impregnated microspheres with aqueous liquid whereby drug is precipitated in the core of the microspheres;
contacting the microspheres containing precipitated drug with an aqueous solution of an ionic drug having a charge opposite to that of the polymer to allow loading of ion-exchange; and
recovering the dual loaded microspheres, wherein the drugs are selected as described above.

In this method, we have found that we can achieve high levels of loading for both the water-insoluble drug and the cationic drug. The level of loading of cationic drug is as high as for the neat microspheres. Where the loading method is carried out with the ionic drug being loaded first and the neutral water-insoluble drug being loaded second, the possible loading levels achieved for the second, neutral drug are lower than for neat microspheres. This effect is further shown in Example 4 below.

We believe it is the first time that a water-swellable ionically charged drug delivery polymer has been loaded with a combination of a water-insoluble drug and a drug having an opposite charge to the polymer. According to a further aspect (referred to hereinafter as the second aspect) of the invention a drug delivery matrix which comprises a water-insoluble, water-swellable ionically charged polymer which is swellable in water at room temperature to an equilibrium

water content in the range 40 to 99% by weight based on polymer plus water is contacted with a solution of drug having a water-solubility less than 10 g/l at room temperature in an organic solvent which is capable of swelling the polymer, whereby the solution becomes impregnated into the matrix;
any drug solution which has not impregnated the polymer matrix is separated;
the impregnated matrix is contacted with water whereby drug is precipitated in the matrix;
the matrix having precipitated water-insoluble drug is contacted with an aqueous solution of the ionically charged drug whereby the matrix swells and ion-exchange with counterions associated with the polymer takes place and drug is electrostatically bound in the polymer matrix.

Optionally the matrix loaded with the low solubility drug is subsequently rinsed and/or dried. The subsequent steps up to contact with the aqueous solution of the ionically charged drug may be as described in WO2007090897 and the preferred components and conditions described in that specification may be used for the first steps in the process of this embodiment, including the drugs. The matrix may be in the form of a microsphere according to the first aspect of the invention. Alternatively it may be a drug delivery device article such as an implant in the form of a needle, slab, disc, thread or film of polymer. Alternatively it is a coating on a device which is implanted such as a stent, catheter, needle, tubing, graft, valve, wire, etc.

The polymer may be an anionic polymer as used in the first aspect of the invention described above. Alternatively it may be a cationic polymer, for instance also having zwitterionic pendant groups, as described in WO0152915. Alternatively it may be another ionic hydrogel polymer used in drug delivery device such as a dextran sulphate-based material as described in Liu *et al.,* (2003) *op. cit.*.

Preferably it is synthetic, although it may alternatively be a natural-based polymer, based on a protein or a polysaccharide, such as collagen, hyaluronic acid, dextran sulphate, alginate, a cellulosic polymer or albumin.

In the method of the second aspect of the invention the matrix loaded with the first drug may be dried before contact with the aqueous solution of the second drug. After ion-exchange with the second drug excess drug solution may be removed before rinsing. This aspect of the invention is of particular value where the water-insoluble drug is rapamycin or an analogue as described above, or a taxane such as paclitaxel, or dexamethasone.

In an alternative embodiment of loading method to produce the product of the first aspect of the invention, the active which is not charged may be a material which is crystalline at room temperature, but for which it would be desirable to avoid being in crystalline form. Such drugs include, for instance, ibuprofen. For these drugs, crystallisation of the drug may be inhibited by incorporating a crystallisation inhibitor, such as an oil, for instance a mineral oil or a vegetable oil, optionally an oil which acts as an imaging agent, for instance lipiodol or another iodinated oil. Suitably the process involves contacting microspheres comprising a matrix of a water-insoluble water-swellable ionically charged crosslinked polymer with a solution of the crystallisable drug in an organic solvent capable of penetrating the microspheres, the solution further containing a crystal modifier which inhibits crystallisation of the drug, and, in a subsequent step, contacting the microspheres loaded with crystallisable drug with an aqueous solution of an ionic drug with a charge opposite that of the polymer matrix in an amount and for a time suitable for loading the drug by ion-exchange. Preferably in the method, following loading of the crystallisable drug, the beads are washed with water or an aqueous solution to remove non-absorbed crystallisable drug.

In the invention of the first or second aspects of the invention, the microspheres may be suitable for administration in an embolisation method. The microspheres are therefore of a size which renders them suitable for use in embolisation, depending on the animal being treated, and the location of the tumour, and thus the size of the feeding blood vessels. For instance embolic microspheres preferably have a size when swollen in deionised water at room temperature (neat) of 40 $\mu$m up to 1500 $\mu$m, preferably in the range 100 $\mu$m up to 1200 $\mu$m. Alternatively the microspheres may be suitable for administration as a drug depot, for instance which may release drug over a long period into the surrounding tissue, and optionally into the circulation. In another embodiment, the microspheres are suitable for intratumoural injection. Microspheres having sizes in the range mentioned above for embolisation may also be suitable for intratumoural administration or for administration in the resection margins following tumour resection.

Methods for loading cationically charged and water-insoluble actives into anionic matrix polymers as described above for use with microspheres, may also be applicable for loading such drug types into other hydrogel based drug delivery articles. In the second aspect of the invention the two drugs are preferably selected so as to be therapeutically effective in tumour treatment, and preferably have a combination index as hereinbefore defined of less than 0.9, preferably less than 0.7, for instance less than 0.5, preferably less than 0.3, and most preferably less than 0.1.

The ionic drug in the second aspect of the invention may be a cationic anthracycline as described in WO04071495, or a camptothecin derivative such as described in WO2006027567. Alternatively the drug may be anionic such as described in WO0152915.

The present invention is illustrated in the accompanying examples. The examples refer to various figures which relate briefly to the following results:

Figure 1 shows the loading profile of beads loaded with doxorubicin (Sample 1) or irinotecan (Sample 2) at a level

of 25 $mg.ml^{-1}$ of beads after loading with the second drug at a saturation level, n=1, as described in Example 2;

Figure 2 shows the loading profile of irinotecan and doxorubicin combined into beads. Loading profile of beads loaded with 25 mg of irinotecan for 1 h and then with 25 mg of doxorubicin (Sample 3), or with 25 mg of doxorubicin for 1 h and then with 25 mg of irinotecan (Sample 4) and loading profile of beads loaded with a mixture of 25 mg of doxorubicin and 25 mg of irinotecan (Sample 5), n=1, as described in Example 2;

Figure 3 shows the size of samples loaded with a combination of doxorubicin and irinotecan by different methods, n=1, as described in Example 2;

Figure 4 shows the elution profile of doxorubicin and irinotecan eluted into PBS at RT from the same beads. Sample 1 is beads loaded with doxorubicin at a level of 25 $mg.ml^{-1}$ of beads after loading with irinotecan at a saturation level, Sample 2 is beads loaded with irinotecan at a level of 25 mg $ml^{-1}$ of beads after loading with doxorubicin at a saturation level. Sample 3 is beads loaded with 25 mg of irinotecan for 1 h and then with 25 mg of doxorubicin, Sample 4 is beads loaded with 25 mg of doxorubicin for 1 h and then with 25 mg of irinotecan and Sample 5 is beads loaded with a mixture of 25 mg of doxorubicin and 25 mg of irinotecan, n=1, as described in Example 2;

Figure 5 shows the release profile of microspheres loaded with doxorubicin and ibuprofen into 200 ml of PBS at RT onto a roller-mixer for 24 h, n=1, as described in Example 3;

Figure 6 is a flow diagram showing two methods of rapamycin and doxorubicin loading into beads, as described in Example 4;

Figure 7 shows the size distribution of Unloaded and drug-loaded Beads, as described in Example 4;

Figures 8A-D show doxorubicin and rapamycin elution from loaded beads to 200 mL PBS, 25 °C. (A) Drug elution from the beads made by Method 1. (B) Elution data of (A) presented as percentage. (C) Drug elution from the beads made by Method 2. (D) Elution data of (C) presented as percentage. In (A) and (C), the amount of drug eluted were corrected to per volume of beads without drug, as described in Example 4;

Figure 9 shows the procedure of ibuprofen-irinotecan bead preparation, as described in Example 5;

Figure 10 shows the size distribution of ibuprofen- and ibuprofen-irinotecan-loaded beads, as described in Example 5;

Figure 11 shows the elution of irinotecan from beads with the combination of ibuprofen and irinotecan. Elution condition: 0.24 ml beads (loaded), 200 mL PBS, 25 °C, as described in Example 5;

Figure 12 shows ibuprofen elution from beads with the combination of ibuprofen and irinotecan. Elution condition: 0.24 mL beads (loaded), 200 ml PBS, 25 °C, as described in Example 5;

Figure 13 shows doxorubicin elution from Beads vs. elution time. (Error bars: 1SD, n=6), as described in Example 6;

Figure 14 shows the viable HepG2 cells percentage as compared to untreated cells with respect to exposure time and bead number from LDH assay. (Error bars: 1SD, n=6), as described in Example 6;

Figure 15 shows the percentage of viable HepG2 cells for rapamycin, doxorubicin and combination (Error bars: 1SD, n=5), results from LDH assay, as described in Example 7;

Figure 16 shows the percentage of viable HepG2 cells for rapamycin, doxorubicin and combination - scale expansion (Error bars: 1SD, n=5), results from LDH assay, as described in Example 7;

Figure 17 shows the percentage of viable HepG2 cells for rapamycin, doxorubicin and combination (Error bars: 1SEM, n=3), results from MTS assay, as described in Example 7;

Figure 18 shows the percentage of viable HepG2 cells for rapamycin, doxorubicin and combination- scale expansion (Error bars: 1SEM, n=3) Results from MTS assay, as described in Example 7;

Figure 19 shows cell viability % (log scale) of HepG2 cells after 72hrs exposure to drug loaded microsphere combinations (MTS assay, n=6, ±SD), as described in Example 8;

Figures 20 to 22 shows the percentage of viable PSN1 cells after 24, 48 and 72 hours (respectively) exposure to mono and dual loaded drug eluting beads (n=7 +/-SD) as described in Example 9;

Figures 23 to 25 show the percentage of viable HepG2 cells after 24, 48 and 72 hours (respectively) exposure to mono and dual loaded drug eluting beads (n=4 +/-SD) as described in Example 9; and

Figures 26 to 28 show the percentage of viable HepG2 cells after 24, 48 and 72 hours (respectively) exposure to mono and dual loaded drug eluting beads (n=4 +/-SD) as described in Example 10.

## Example 1: Preparation of Drug Eluting Beads

Microspheres are synthesised as described in Reference Example of WO2006027567 to produce low AMPS and high AMPS formulations.

## Example 2: Loading and Elution of Irinotecan and Doxorubicin Combinations

Five samples of 500-700 μm High AMPS beads of Example 1 were loaded with doxorubicin and irinotecan following different approaches:

Sample 1: 3.5 mL of irinotecan solution (Campto, Pfizer, 20 mg.mL$^{-1}$= 70 mg) was loaded into 1 mL of beads for 48 h, to saturate all the sulfonate groups. Then, the depleted solution was removed and the loaded beads were washed with a known volume of deionised water to remove the non-bound drug. The amount of irinotecan loaded into the beads was calculated by depletion, as a difference between the initial and final amounts in the depleted solution and all washings measured by UV at a wavelength of 369 nm. One mL of doxorubicin (Dabur oncology-25 mg.ml$^{-1}$) was later added to the same beads and the loading profile determined.

Sample 2: Similar to sample 1, but 2.8 mL of doxorubicin (25 mg.ml$^{-1}$ = 70 mg) solution was added to the beads instead of the irinotecan solution in the first step, to saturate all the sulfonate groups. All solutions were measured by UV at a wavelength of 483 nm wavelength and the amount of drug loaded into the beads determined by depletion. After the beads were loaded and washed with deionised water, in a second step, 1.25 ml of irinotecan solution (20 mg.mL$^{-1}$ = 25 mg) was added to the same beads and the loading profile determined.

Sample 3: 1.25 mL of irinotecan solution (20 mg.ml$^{-1}$ = 25mg) was loaded into 1 ml of beads and after 1 h, 1 ml of doxorubicin (25 mg.ml$^{-1}$) was added to the same beads. No intermediate washes were performed in this or the following samples.

Sample 4: 1 ml of doxorubicin (25 mg.ml$^{-1}$) was loaded into 1 ml of beads and after 1 h 1.25 mL of irinotecan solution (20 mg.ml$^{-1}$ = 25mg) was added to the same beads.

Sample 5: 1.25 ml of irinotecan solution (20 mg.ml$^{-1}$ = 25mg) was mixed with 1 ml of doxorubicin (25 mg.ml$^{-1}$) and then added to 1 ml of beads.

To determine the loading profiles of all samples, an aliquot of 50 μl was removed from each vial at time 5, 15, 30, 45 min after the addition of each drug, diluted as necessary and read by UV at 483 and 369 nm. The amount loaded at each time point was determined by depletion using the corresponding standard curves.

After loading with both drugs, all samples were sized in their loading solutions using the Colorview III camcorder and pictures were taken. For that, at least 100 beads were measured. These data were normalised and the corresponding histograms generated.

After being sized, all samples were eluted. Here, the remaining loading solutions were removed using a Pasteur pipette and the loaded beads were transferred to brown flasks containing 200 ml of PBS (Inverclyde Biologicals). At time 5, 15, 30, 45, 60, 90 min, 2 h, 4 h and 24 h, an aliquot of elution medium (4 ml) was removed and replaced with 4 mL of fresh PBS to maintain the volume. All samples were read by UV at 483 and 369 nm wavelengths, using the corresponding standard curves to calculate the amount eluted at each time point.

Calculations:

To calculate the loading and elution of both drugs from the same beads the following assumptions were made: Absorbance at 483 nm = Absorbance of doxorubicin at 483 nm.

It is observed in the UV scan of irinotecan solution that this drug does not absorb at this wavelength.

Absorbance 369 nm = Absorbance of doxorubicin at 369 nm + Absorbance of irinotecan at 369 nm.

The doxorubicin scan showed some absorbance at 369 nm wavelength and it is considered here that there is not interaction between the absorbance of doxorubicin and irinotecan when measured at the same time. The scan from the mixture does not show any notable interaction between the absorbance spectra of both drugs.

These two assumptions were made for all calculations. All samples were read at both 483 nm and 369 nm wavelengths. Using the absorbance and the standard curve of doxorubicin at 483 nm, the concentration of doxorubicin in each sample was calculated and using these concentrations and the standard curve of doxorubicin at 369 nm, the absorbance of doxorubicin at this wavelength was calculated. Then, subtracting this value from the total absorbance at 369 nm, the absorbance at 369 nm and hence the concentration of irinotecan was calculated.

Loading of Irinotecan and Doxorubicin into the same Beads

The objective of these experiments was to assess and compare the loading and elution behaviour of different combinations of doxorubicin and irinotecan in order to determine their mechanism of interaction with the beads and evaluate possible interactions between drugs. If both drugs could be successfully incorporated in the same beads it would be simple to achieve the local delivery of both antitumour drugs simultaneously if required.

Doxorubicin and irinotecan were loaded following the different approaches detailed above and results are presented as follows:

As seen in Figure 1, if irinotecan is loaded at maximum bound capacity first and then doxorubicin is added to the system (Sample 1), some irinotecan (~ 17%) is displaced, but the majority of drug is retained by the beads. If doxorubicin is loaded at maximum bound capacity first and then irinotecan is added to the system (Sample 2), a smaller percentage of doxorubicin (< 8%) is displaced by the irinotecan. These data agree with the fact that the $K_b$ values for doxorubicin are slightly higher than those calculated for irinotecan (Gonzalez et al 2006) and doxorubicin releases much slower and to a lower extent than irinotecan in the same volume of solution. In both cases, however, the amount of drug displaced is small, indicating both interactions are stable after addition of the second drug. This could be explained as a consequence of the small volumes of solution used, easily saturated, in the loading process and the initially-bound bulky drug molecule sterically hindering the approach of the second drug to the binding sites, a phenomenon that would not be experienced by small ions, such as $Na^+$ or $K^+$. These two factors will contribute to the small percentage of drug displaced by the second drug added.

Sample 3 and 4 in Figure 2 show that if the beads are first loaded with 25 mg of one drug and then 25 mg of the other drug is added, very little of the bound drug (< 5%) is displaced on addition of the second drug, independent of the order. These results are expected, as the maximum loading capacity of the beads is ~ 40 mg and 52 mg for doxorubicin and irinotecan respectively and the binding sites of the beads are not saturated when the second drug is added (initially, 25 mg of drug are added to the beads and then 25 mg of the other drug is added).

Sample 5 on Figure 2 shows that if both drugs are loaded at the same time, the loading rate and the total amount loaded is slightly higher for doxorubicin than for irinotecan (94% of the total doxorubicin added (25 mg) is loaded whereas 91% of the total irinotecan added (25 mg) is loaded after 2 h). This small difference could be explained as a result of their $K_b$ values, where doxorubicin $K_b$ value ($5.58*10^8$-$1.02*10^9$) is slightly higher than for irinotecan ($8.73*10^7$-$3.62*10^8$).

<u>Sizing of Irinotecan and Doxorubicin Loaded into the Same Beads</u>

No notable differences were detected between beads loaded with both drugs by the different approaches (Figure 3).

<u>Elution of Irinotecan and Doxorubicin Loaded into the Same Beads</u>

Figure 4 shows that all samples follow similar elution trends and doxorubicin is released very slowly (< 25% in 24 h) compared to irinotecan (> 99% mg in < 4 h) when eluted into 200 ml of PBS.

In samples 1 and 2, where one drug was loaded up to saturation level before the other drug was added, the second drug to be loaded was released faster than in sample 3 and 4, where both drugs were loaded into the beads at the same time below saturation level. This could be explained by the partially bound nature of the second drug to be loaded, caused by all the sulfonate groups being saturated (sample 1 and 2). A small amount of the drug loaded second would be bound to the beads by displacing the first, mostly bound drug. Most of the loaded drug would be free (unbound) inside the beads and would elute faster than the bound drug.

In conclusion, when doxorubicin and irinotecan are loaded at the same time or consecutively into the beads, no change in the release behaviour of the other drug is observed. The presence of an additional drug does not interfere the loading or modify the release behaviour of the other drug. Each drug elutes as expected from the individual drug elution profiles, generated for each drug alone.

UV profiles of the eluted solutions were performed and compared to an unloaded mixture of doxorubicin and irinotecan. The shape of the peaks and the maximum values did not change, confirming that no apparent interaction or modification of either drug occurs inside the beads.

These experiments demonstrate that is possible load and elute both drugs at the same time from the same beads. Sustained release of the two drugs is expected from this product. The combination of these two anti-tumoural drugs is expected to achieve higher therapeutic effect than the treatment with a high-dose single drug and to decrease the adverse effects of both drugs. This potential product will be evaluated in vivo to determine their effect as synergistic, additive or antagonistic. This effect will depend on the molecular ratios of individual chemotherapy agents and schedules of administration (Aschele et al. 1998). In vivo studies will be carried out to evaluate different doses of the two agents in order to determine the best ratio. These experiments revealed that both drugs could be successfully loaded and eluted within the same beads without alter the release profiles of the drugs individually and suggest the possibility of loading more than one cationic drug in the beads.

**<u>Example 3: Combination of Ibuprofen and Doxorubicin in the Same Beads</u>**

Ibuprofen and doxorubicin solutions were loaded into low AMPS beads simultaneously. In order to achieve this, one sample of 1 ml of 500-700 μm beads was loaded with ibuprofen (according to examples in WO2004073688).

The excess packaging solution was removed using a Pasteur pipette and 2 ml of doxorubicin solution (5 $mg.ml^{-1}$) were added. After 24 h, a 50 μl aliquot was removed from the depleted solution and measured by UV at 263 nm and 483 nm. The amount of doxorubicin loaded into beads was calculated by depletion, as a difference between the initial and final amounts in the depleted solution (calculations explained below).

After loading with both drugs, the depleted loading solution was removed and the loaded beads were transferred to brown flasks containing 200 mL of PBS. At time 5, 45, 90 min, 3, 5 and 24 h, 2 ml of elution medium was removed and replaced with 2 ml of fresh PBS to maintain the volume. All samples were read by UV at 263 and 483 nm and the amount of each drug eluted at each time point was calculated using the corresponding standard curves.

Calculations:

To calculate the loading and elution of both drugs from the same beads the following assumptions were made: Absorbance at 483 nm = Absorbance of doxorubicin at 483 nm.

The UV scans of ibuprofen and doxorubicin solution showed that only doxorubicin absorbs at this wavelength.

Absorbance 263 nm = Absorbance of doxorubicin at 263 nm + Absorbance of ibuprofen at 263 nm.

The doxorubicin UV scan showed some absorbance at 263 nm wavelength. The assumption is made that there is no interaction between the absorbance of doxorubicin and ibuprofen when measured at the same time.

These two assumptions were made for all calculations. All samples were read at both wavelengths (263 nm and 483 nm). Using the absorbance at 483 nm the concentration of doxorubicin in each sample was calculated, then using these concentrations and the standard curve of doxorubicin at 263 nm, the absorbance of doxorubicin at 263 nm was calculated. By subtracting this value from the total absorbance at 263 nm, the concentration of ibuprofen, was calculated using the ibuprofen standard curve.

Loading and Release of Doxorubicin and Ibuprofen Combinations in Beads

In the first set of experiments, doxorubicin and ibuprofen were combined into the same beads and their release behaviour was evaluated.

Initially, lyophilised beads as described in WO2004073688 were loaded with ibuprofen /ethanol solution by solvent evaporation and then rehydrated with water and autoclaved for 15 min at 121°C. The rehydrated ibuprofen loaded beads were then loaded with doxorubicin by immersion into doxorubicin solution (5 $mg.ml^{-1}$) for 24 h. In this experiment, a maximum amount of 8.3 mg of doxorubicin was loaded into the beads calculated by depletion of the concentration of the loading solution. The low AMPS beads have a maximum capacity for doxorubicin of -14 $mg.ml^{-1}$ when loaded with this drug alone. This maximum amount is limited by the number of binding sites available in the beads. If a greater amount of drug were to be added to the sample, it would not be loaded as all the sulfonate groups within the beads would have been saturated.

The release of both drugs from the same beads was monitored by UV spectrophotometry at the same time, the elution profile for both drugs released is presented in Figure 5. Both drugs had similar release profiles to those exhibited for beads loaded with only one of the drugs and no interaction was observed between the two drugs. This experiment showed that both drugs can be loaded and released into the same bead, offering a platform for sustained released of two drugs simultaneously. This could be beneficial as a drug delivery device by adding the therapeutic effects of both drugs by administration of one product. This therapy could reduce the requirements of analgesic drugs following the procedures, and the dose of the more toxic drug if synergistic antitumoral results by the combination of both drugs are demonstrated.

**Example 4: Combination of Doxorubicin and Rapamycin in the Same Beads**

Loading methods: Two loading methods were tested as shown in Figure 6).

Method 1

Unloaded high AMPS bead slurry 1 ml was loaded with doxorubicin solution (5 ml of 10.07 mg/ml, target loading 50 mg/ml Bead) overnight. The doxorubicin-loaded beads were washed with 1 ml DMSO 5 times to remove extra water, and 1 ml 60 mg/mL rapamycin DMSO solution was mixed with the doxorubicin beads for about 30 min. Subsequently, following removal of extra rapamycin DMSO solution, the beads were washed with 5 ml saline until no white drug particles

were observed.

Method 2

1 ml Bead slurry was washed with 1 mL DMSO 5 times and mixed with 1 ml 60 mg/ml rapamycin DMSO solution for 30 min. The loaded beads were then washed with 5 ml saline until no further white drug particles were observed. 0.5 ml of rapamycin-loaded bead slurry was roller-mixed with 2.5 ml 10.07 mg/ml doxorubicin solution overnight, target doxorubicin loading is 50 mg/ml.

Doxorubicin loading test: The doxorubicin loading was measured by depletion method in either water or DMSO solution. The loading solution was taken and diluted 10 times and measured by UV at 483 nm to determine the concentration of doxorubicin residue in solution.

Rapamycin loading test: For the beads with rapamycin alone, 0.5 ml rapamycin-loaded beads (from 1st step, Method 1) were extracted by 1 ml DMSO 5 times, and the solution were accumulated in a 10 ml volumetric flask. The concentration of rapamycin was determined by UV at 279 nm.

For the beads with doxorubicin and rapamycin combination, 0.26 ml of drug-loaded beads (from 2nd step, Method 2) were extracted with 1 ml DMSO 4 times, and the extraction was collected in a 5 ml volumetric flask and DMSO was added to top the solution to the mark. The concentration of rapamycin was determined by subtraction of the absorbance of doxorubicin at 279 nm from the total absorbance at 279 nm, in which the doxorubicin absorbance at 279 nm was calculated from that at 483 nm according to the scan of concentration-known solutions.

Doxorubicin loss determination: The doxorubicin loss during DMSO washing of loaded beads before further loading of rapamycin (Method 2) was determined by measuring the accumulated DMSO washing solution in a volumetric flask at 483 nm.

Elution of drug-loaded beads: The elution of drug-loaded beads were carried out by mixing the beads with 200 ml PBS (Inverclyde, Bellshill, UK) and roller-mixed on a roller mixer. At certain time point, the solution (100 ml or 150 ml) was taken out for concentration determination by UV and same volume of fresh PBS was added to keep a sink condition. The determination rapamycin concentration followed the same principle as that in the section of Rapamycin Loading Test.

Image analysis and sizing: The loaded beads were placed under an Olympus microscope, about 200 beads were counted for size distribution study by software (AnalySIS 5.0 Soft Image System GmbH), and photos were taken under different light/contrast background for morphology study.

Rapamycin and doxorubicin standard curve: The maximum absorbance of rapamycin is at wavelength 279 nm, which is overlapped with doxorubicin absorbance. At 483 nm, the wavelength of maximum absorbance of doxorubicin, rapamycin has no absorption. Therefore, the rapamycin concentration in a bicomponent system (doxorubicin and rapamycin) could be determined by first measurement of standard solutions of doxorubicin at both wavelength, then subtraction of the absorbance of doxorubicin from the absorbance of mixture. Standard curves are created of doxorubicin at 279 and 483 nm in water and DMSO.

The concentration of rapamycin after subtraction of doxorubicin absorbance can be determined by the relationship given underneath at 279 nm:

$$\text{Absorbance} = 56.18 \times \text{Concentration (mg/ml)} \qquad \text{[in DMSO]}$$

$$\text{Absorbance} = 44.64 \times \text{Concentration (mg/ml)} \qquad \text{[in water]}$$

Comparison of drug loading in the two loading procedures

In the 1st step of Method 1, rapamycin was loaded at 28.48 mg/ml, the loading efficiency was about 47.5% (Table 1). Compared to the rapamycin loading in the 2nd step of Method 2, 37.22 mg/ml rapamycin was loaded, the loading efficiency being 62.0%. This significantly high loading efficiency was consistent with the observation that the doxorubicin-loaded beads swelled larger and most of the rapamycin loading solution was sucked into the beads. From a mechanism point of view, the doxorubicin-loaded beads have smaller size, and tend to swell larger in polar solvents like water and DMSO due to the reduced salt effect which was observed in doxorubicin loading solution or saline.

For the beads loaded with rapamycin (28.48 mg/ml), the doxorubicin loading capability was found to be 46.02 mg/ml, similar to that of beads without rapamycin. During doxorubicin loading, no rapamycin leakage was observed. This experiment suggests that rapamycin preloading does not affect the later doxorubicin loading, which means that the two drugs occupy different sites within the beads. Doxorubicin molecules tend to bind to the site of the sulfonate groups by ionic interaction; however, rapamycin stays within the interstitial space within the hydrogel by expelling water out and forming small drug crystals.

**Table 1.** Rapamycin and doxorubicin loading by two methods[a]

| Sample list | Doxorubicin loading (mg/ml [b]) | Rapamycin loading (mg/ml [b]) |
|---|---|---|
| Rapamycin loading in 1st step, Method 1 | - | 28.48 |
| Doxorubicin loading in 1st step, Method 2 | 45.88 | - |
| Doxorubicin loading in 2nd step, Method 1 | 46.02 | 28.48 [c] |
| Rapamycin loading in 2nd step, Method 2 | 39.17 [d] | 37.22 |

[a] The data shown in the table are average values of two replicates.
[b] All the drug loading data were normalised to the volume of beads without drug loading.
[c] No rapamycin loss was observed during doxorubicin loading, therefore the same data as that in 1st step was used.
[d] The doxorubicin loss during DMSO washing in preloading of rapamycin was 6.71 mg/ml. The further doxorubicin loss during rapamycin loading was ignored.

In the Method 2, the washing of doxorubicin-loaded beads with DMSO before rapamycin loading caused doxorubicin loss of about 6.71 mg/ml, although this method had a higher rapamycin loading. The final doxorubicin to rapamycin ratio was 1.62 for beads from Method 1, and 1.05 for beads from Method 2.

From an operational point of view, Method 1 is a relatively clean procedure and only produces rapamycin waste solution. Method 2 produces both doxorubicin-DMSO and rapamycin waste solution. Doxorubicin loading in Method 1 is almost completed, and Method 1 is also relatively easier to process. In spite of high loading of rapamycin in Method 2, Method 1 is recommended.

Image analysis and size distribution of drug-loaded beads

Photographs are taken after rapamycin and rapamycin-doxorubicin loading by Method 1. It appears that these beads retain their smooth surface and spherical shape well after drug loading. Some small particles of rapamycin were observed, which are the residues from the 1st step. The doxorubicin-loaded beads experienced a slight lost of their spherical shape. After additional loading with rapamycin, the shape recovered well and their surfaces were smooth. If compared to the final beads from Method 1, the beads from Method 2 have shallow red colour, which may be due to doxorubicin loss after DMSO washing, or due to later loaded rapamycin coverage.

The size characterisation of DC Bead and drug-loaded beads were listed in Table 2, and the size distribution was shown in Figure 7. From Table 2, the rapamycin-loaded beads in Method 1 have a slight increased sizes compared to unloaded beads. Once loaded with doxorubicin, the mean size decreased by about 100 $\mu$m. In Method 2, the doxorubicin-loaded beads show the smallest mean size. After loading with rapamycin, the size increased to about 479 $\mu$m, close to the final bead size from Method 1. Meanwhile, the standard deviation data in Table 2 indicate that the range of size distribution has almost no change during drug loading. Therefore, the two methods give final beads having almost the same size range (Figure 7).

**Table 2.** Size characterisation of Unloaded and drug-loaded beads

| Sample list | Maximum size ($\mu$m) | Minimum size ($\mu$m) | Mean size ($\mu$m) | Standard deviation ($\mu$m) |
|---|---|---|---|---|
| Unloaded Beads | 645 | 451 | 546 | 46 |
| After rapamycin loading in 1st step, Method 1 | 662 | 471 | 560 | 48 |
| After doxorubicin loading in 1st step, Method 2 | 518 | 365 | 444 | 39 |
| After doxorubicin loading in 2nd step, Method 1 | 532 | 360 | 466 | 36 |
| After rapamycin loading in 2nd step, Method 2 | 562 | 402 | 479 | 40 |

Drug elution from loaded beads

Figure 8 shows the elution data of doxorubicin and rapamycin from loaded beads into PBS. In this experiment, the elution medium was refreshed after a certain time to mimic sink conditions. From Figure 8 (B) and (D), the drug elution profiles from the beads made by the different methods are very similar: rapamycin released about 33% after 30

hr elution, and doxorubicin released about 41 to 46%.

Comparing Figure 8 (A) and (C), the doxorubicin from beads made by Method 1 eluted slightly faster than the beads made by Method 2. For rapamycin elution, the reverse phenomenon was observed. This may be due to the later loaded drug covered or blocked the pathway of the first drug, which was also used for the first drug elution.

**Example 5: Combination of Irinotecan and Ibuprofen in the Same Bead**

Drug loading: The drug loading procedure is shown in Figure 9, in which the unloaded high AMPS beads were washed with DMSO to remove the water trapped within the hydrogel. A mixture of ibuprofen solubilised in DMSO and castor oil as crystallisation inhibitor were subsequently mixed with the bead slurry, and kept for about 10 minutes. Following the removal of the loading solution, saline was used to wash the loaded beads until no drug particles nor large oil droplets were observed. The irinotecan loading was achieved by simply mixing the ibuprofen-loaded beads with irinotecan solution (10 mg/mL) by roller-mixing overnight. The final loaded beads were kept in the water solution as product.

The sample matrix of ibuprofen-irinotecan beads was listed in Table 3.

**Table 3.** Sample matrix of ibuprofen-irinotecan loading

| Sample | Composition of ibuprofen loading solution | | | Irinotecan loading solution (10.07 mg/mL) |
|---|---|---|---|---|
| | Ibuprofen (g) | DMSO (ml) | Castor oil (g) | Volume of Irinotecan solution (ml) |
| Ex 5-1 | 0.8 | 5 | 5 | 4 |
| Ex 5-2 | 0.4 | 5 | 5 | 4 |
| Ex 5-3 | 0.2 | 5 | 5 | 4 |
| Ex 5-4 | 0.8 | 2.5 | 7.5 | 4 |

Determination of drug content in beads:

1). Ibuprofen. The content of loaded ibuprofen was determined by an HPLC method. The slurry of drug-loaded beads with known volume was extracted with DMSO (1 ml) for five to six times. The collected DMSO solution was topped to the mark in a 10 ml volumetric flask, and the concentration of ibuprofen was measured by HPLC.

2). Irinotecan. The content of irinotecan in loaded beads was measured by depletion method. 1 - 1.1 ml of ibuprofen-loaded beads were roller-mixed overnight with 4 mL 10 mg/ml irinotecan solution to target the loading at 40 mg/ml beads. The loading solution was diluted with water and measured by UV at 369 nm. Compared to the standard curve, the concentration of irinotecan left in loading solution can be calculated.

Elution of drug-loaded beads:

The elution of ibuprofen-irinotecan-loaded beads was carried out by mixing 0.24 ml beads with 200 ml PBS (Inverclyde, Bellshill, UK) and roller-mixed on a roller mixer. At certain time point, the solution (5 ml) was taken out for concentration determination by UV and same volume of fresh PBS was added to keep a sink condition.

Image analysis and sizing:

The loaded beads were placed under an Olympus microscope, about 200 beads were counted for size distribution study by software (AnalySIS 5.0 Soft Image System GmbH), and photos were taken under different light/contrast background for morphology study.

Determination of concentration of combination of ibuprofen and irinotecan

To determinate the concentration of each component in a combined drug solution, the standard curves for absorption of predetermined concentrations of ibuprofen and irinotecan at 263, 272 and 369 nm in water were measured. The principle is to determine the concentration of irinotecan from the absorbance at 369 nm first, where ibuprofen has no absorption. Then the concentration of ibuprofen could be calculated by subtracting the irinotecan absorbance at 272 nm from the spectrum of drug mixture. Using 272 nm instead of 263 nm commonly used is to reduce the possible interference of DMSO residue in drug loading. However, it was found that the ibuprofen absorbance is much lower

compared to the irinotecan due to the low water-solubility of ibuprofen, and the ibuprofen peak was fully covered by irinotecan. Therefore the way using UV to determine ibuprofen concentration in ibuprofen-irinotecan mixture is not accurate. In this study, the irinotecan concentration was measured by UV, and ibuprofen concentration was measured by HPLC.

Loading ibuprofen and irinotecan into Beads

All the samples loaded with ibuprofen in the first step retain their spherical shape and no ibuprofen crystals were seen to form. After irinotecan loading, Example 5-1 with high ibuprofen loading had a large amount of ibuprofen crystals appear on the bead surface. In comparison, the Example 5-4 with the same amount of ibuprofen has no crystals observed, which is due to the effect of the high content of castor oil crystallisation inhibitor existing within beads. Therefore, the castor oil acts as an excipient to prevent the formation of ibuprofen crystals by inhibiting crystal formation.

Table 4 lists the drug loading in each sample. The irinotecan loading in all the samples is close to 100%, the maximum irinotecan loading capacity for unloaded beads being close to 50-60 mg/ml, and below that the loading efficiency is above 98%.

**Table 4.** Drug loading in Unloaded Beads

| Sample | Target ibuprofen loading* (mg/ml) | Actual ibuprofen loading (mg/ml) | Target irinotecan loading (mg/ml) | Actual irinotecan loading (mg/ml) |
|---|---|---|---|---|
| Ex 5-1 | 40 | 14.34** | 40.28 | 40.28 |
| Ex 5-2 | 20 | 10.41 | 40.28 | 40.28 |
| Ex 5-3 | 10 | 5.79 | 40.28 | 40.28 |
| Ex 5-4 | 40 | 11.68 | 40.28 | 40.28 |

* The data were estimated according to the total volume of DMSO and castor oil treated as about 10 ml.
** Including the ibuprofen crystal on bead surface.

The ibuprofen loading is relatively low, and the estimated loading efficiency is between 29% to 58%. The lower target of loading of ibuprofen gives a relatively higher loading efficiency. The reason of the relatively low ibuprofen loading is due to the bead size shrinking when the beads contact with castor oil rich solution. Hence, the drug loading decreased with the smaller volume of beads.

Size distribution of drug-loaded beads

The size distribution of drug-loaded beads is shown in Figure 10. The corresponding parameters are listed in Table 5. The ibuprofen-loaded beads almost keep the same size as the DC Bead without drug loading, in the range from 500 to 700 μm. However, the size decreased significantly with irinotecan loading due to the increased hydrophobicity of the drug-loaded beads, the average size decrease is about 140 to 150 μm. It is noticed that the beads with variance of ibuprofen loading have almost the same mean size and size distribution.

**Table 5.** Comparison of size distribution for drug loaded beads

| Sample | Drug loaded | $Size_{max}$ (μm) | $Size_{min}$ (μm) | $Size_{mean}$ (μm) | SD (μm) |
|---|---|---|---|---|---|
| 849BB/20-1 | IBU* | 690 | 476 | 589 | 53.5 |
| 849BB/20-1 | IBU-IRI** | 549 | 340 | 448 | 41.7 |
| 849BB/20-2 | IBU | 688 | 470 | 592 | 47.4 |
| 849BB/20-2 | IBU-IRI | 510 | 354 | 432 | 33.6 |
| 849BB/20-3 | IBU | 670 | 474 | 585 | 56.0 |
| 849BB/20-3 | IBU-IRI | 532 | 371 | 444 | 43.7 |
| 849BB/20-4 | IBU-IRI | 527 | 362 | 446 | 36.1 |

• IBU: ibuprofen
** IRI: irinotecan

Morphology of drug-loaded beads

A micrograph of ibuprofen-loaded beads of Example 5.1, demonstrates that the structure of the loaded beads consist of a large core area with ibuprofen and castor oil, and a layer on the bead surface free of drug and oil. Castor

oil trapped inside the beads provides the hydrophobic phase to retain ibuprofen and to prevent the formation of ibuprofen crystals. The other Examples 5-2, 5.3 and 5.4 with ibuprofen in the first loading step have the same structure shown here. In addition, some small oil droplets were observed among the beads, which were the residues of mixture of castor oil and ibuprofen in solution.

When irinotecan was loaded by roller-mixing overnight, ibuprofen crystals were observed on the surface of the beads in Example 5.1, and bead aggregation occurred as the result of crystal formation. Crystallisation was not found in other samples. The phenomenon is due to the higher ibuprofen content in the beads, and not enough castor oil to prevent crystal formation, when irinotecan was loaded. Although the irinotecan interacts with the AMPS group in the beads, it still is hydrophobic and may tend to partially dissolve in castor oil, which could reduce the solubility of ibuprofen. In the product of Example 5.4, with a higher level of castor oil and high loading of ibuprofen, after irinotecan loading, no ibuprofen crystals were observed.

Elution of drug-loaded beads

Figures 11 and 12 show the elution profile of irinotecan and ibuprofen from beads into PBS at 25°C. In Figure 11, the elution rate of irinotecan under different castor oil content and ibuprofen loading are almost the same. The irinotecan elution rate is also comparable to that for irinotecan alone in which almost 100% of the drug was eluted after 5 hours. This indicates that castor oil and ibuprofen loading have no effect on irinotecan elution, and there is no interaction between ibuprofen and irinotecan. In Figure 12, the ibuprofen elution is completed after 1 to 2 hr in PBS. Compared to the ibuprofen elution from single drug-loaded beads, the elution again is similar indicating no drug-drug interaction is occurring.

**Example 6: In vitro Cytotoxicity Evaluation of Doxorubicin Loaded Beads using Human HCC Cells (Reference).**

Doxorubicin beads (500-700μm) were prepared according to the methods outlined in Example 2 of WO04071495. The elution of doxorubicin loaded beads in cell culture media with a human hepatocellular liver carcinoma (HepG2) cell line was monitored. A Promega lactate dehydrogenase (LDH) assay was used to assess the cytotoxicity effects on the HepG2 cell line at 24, 48 and 72 hour time points.

Assay preparation

Using aseptic conditions, three sterile flat bottomed 96 well plates (Cellstar®, 655180) were seeded with 20,000 HepG2 cells in 200μl media and left in an incubator (37°C, 5% $CO_2$) for 20 hours. After incubation, the media was carefully aspirated from all of the wells and 200μl of PRF media was added to the wells. One, three and ten pre-loaded doxorubicin beads (37.5 mg per ml of beads) were counted under aseptic conditions into 200μl phenol red free cell culture media in a 96 well plate.

Doxorubicin elution assay

The plate was incubated at 37ºC, 5% $CO_2$. At 24, 48 and 72 hour time points, 100μl of each well were scanned at 490 nm (Biotek, ELX800). The elution was calculated by comparing the absorbance to a standard calibration curve.

At longer time periods and higher bead numbers more doxorubicin was released into the cell culture media (Figure 13). The elution profile is different for different bead numbers; the plateau for 10 beads at later time period may be due to saturation of the cell culture media with doxorubicin.

Cell viability assay

This assay measures the amount of LDH released from the remaining viable cells after the dead cell debris have been washed away. The LDH is released after the viable cell membranes have been compromised by freezing the cells.

At each time point, the media and beads was carefully aspirated from the original plate and 200μl sterile PBS was added and removed three times to wash the cells of any residual doxorubicin and left over cell debris. 200μl PRF media was added before the plate was subjected to -80ºC for one hour; this intentionally compromised all of the cell membranes, releasing all lactate dehydrogenase enzyme from the remaining live cells. Upon removal from the freezer the plate was promptly placed in an incubator under standard conditions (37ºC, 5% $CO_2$) for thirty minutes. 50μl of the supernatant was removed from all the wells into respective fresh wells, followed by 50μl of the LDH assay substrate mix (Promega). The original plate with cells was kept for further analysis. The fresh plate was left a room temperature in the dark. After 30 minutes 50μl of stop solution (acetic acid (1M)) was added to terminate the reaction. The plate was

then read using a plate reader (Biotek) at 490nm, if the reading was too high (>3) the solution was diluted by taking 75µl supernatant and 75µl PRF media. The percentage of compromised cells was calculated.

The number of viable cells decreases as the exposure to and number of pre-loaded doxorubicin DC beads increases (Figure 14).

## Example 7: Cytotoxicity of Combinations of Doxorubicin Beads and Rapamycin Beads and in-vitro

A LDH assay was used to assess the cytotoxicity effects on the HepG2 cell line at 24, 48 and 72 hour time points with different rapamycin/doxorubicin bead combinations.
The rapamycin beads (500-700µm) were loaded and analysed described in PCT/EP2007/50690. The resulting loading was 21.4mg/ml.

The doxorubicin beads (500-700µm) were loaded with 37.5 mg per ml of beads as described in WO04071495.

### Assay preparation

Using aseptic conditions, three sterile flat bottomed 96 well plates (Cellstar®, 655180) were seeded with 20,000 HepG2 cells in 200µl media and left in an incubator (37°C, 5% $CO_2$) for 20 hours.

After incubation, the media was carefully aspirated from all of the wells and 200µl of PRF media was added to the wells.

Pre-loaded doxorubicin beads and pre-loaded rapamycin beads were counted under aseptic conditions into 200µl phenol red free cell culture media in a 96 well plate. The number of each beads placed in each well can be seen in table 5 (n=6). A control of each drug type was carried out alongside the assay (1-5 beads of one drug type only).

**Table 6:** Matrix showing beads ratios

| | Number of beads per well | | | | | | |
|---|---|---|---|---|---|---|---|
| Rapamycin beads | 1 | 1 | 1 | 1 | 2 | 3 | 4 |
| Doxorubicin beads | 1 | 2 | 3 | 4 | 1 | 1 | 1 |

After 72 hours exposure to the cells, an LDH assay as described in Example 6 was carried out on the controls and samples in Table 6.

An MTS assay was also conducted on the wells. At 72hrs, the media from all wells was cautiously removed and each well washed three times with 200µl sterile PBS. After adding 100µl PRF media to each well, 20µl of the prepared MTS solution (Promega) was also added. After a two hours incubation (37°C, 5% $CO_2$) the plate was read at 490nm using the plate reader and like the LDH assay, if the reading was too high the samples were diluted (75µl PRF media: 75µl samples).

### Synergy of rapamycin and doxorubicin

The cell viability experiment with rapamycin beads and doxorubicin beads in different ratios allow any drug synergy to be established. A single rapamycin bead and a single doxorubicin bead together in one well left fewer viable cells than two beads of one drug type. This trend is seen throughout the bead combinations tested (Figure 15 & 16). A similar trend is noted with the MTS assay (Figures 17 & 18).

Using Calcusyn v2.1 (Biosoft) a combination index value can be established for each combination using the data from the LDH assay (Table 7). The value of the combination index relates to a scale (Table 7). Using these descriptions a strong synergism is noted under these conditions.

**Table 6:** Combination Index (CI) Values and description. (Fa = fraction affected by the dose, 1= 100% kill, 0= no kill).

| **Rapamycin(beads)** | **Doxorubicin(beads)** | **Fa** | **CI** |
|---|---|---|---|
| 1 | 1 | 0.984 | 0.007 |
| 1 | 2 | 0.991 | 0.002 |
| 1 | 3 | 0.998 | 1.06e-5 |
| 1 | 4 | 0.994 | 0.001 |
| 2 | 1 | 0.997 | 1.52e-0.005 |
| 3 | 1 | 0.988 | 0.002 |

**Table 7:** Combination Index Values and description

| Range of CI | Description |
|---|---|
| <0.1 | +++++ Very strong synergism |
| 0.1-0.3 | ++++ Strong synergism |
| 0.3-0.7 | +++ Synergism |
| 0.7-0.85 | ++ Moderate synergism |
| 0.85-0.90 | + Slight synergism |
| 0.90-1.10 | ± Nearly additive |
| 1.10-1.20 | - Slight antagonism |
| 1.20-1.45 | - - Moderate antagonism |

As can be seen all doxorubicin-rapamycin combinations gave a CI that indicated a very strong synergism between the two drugs. Both assays show synergy between both drugs. The data do not show a preferred ratio, but synergy is shown for all combinations.

**Example 8: *In Vitro* Cytotoxicity of Rapamycin and Doxorubicin Loaded Beads Individually and in Combination**

Example 7 was repeated substantially as described, with an extended number of beads of one of the drugs. The results of the MTS assay, i.e. showing cell viability % (log scale) of HepG2 cells after 72 hr exposure, concluded as the mean of 6 samples, is shown in Figure 19.

**Results**

When eluted individually, doxorubicin was more cytotoxic to the cells than rapamycin (Figure 1). However, when used in combination, a further reduction in cell viability is observed. The combination indices for all bead combinations are <0.1; this indicates strong positive synergy between the two eluted drugs.

**Example 9.: Synergy of Rapamycin and Doxorubicin on Human Pancreatic Carcinoma (PSN1) and Human Hepatocellular Carcinoma (HepG2) Cell Lines *in-vitro***

An MTS assay was used to assess the synergistic cytotoxic effects of dual loaded beads on HepG2 and PSN1 cells. The rapamycin loaded beads and the doxorubicin loaded beads were the same as those described in Example 7. The dual loaded doxorubicin and rapamycin loaded beads were loaded and analysed as described in Example 4 Method 2 using a 1ml 60mg/ml rapamycin solution and 1ml 37.5mg/ml doxorubicin solution. The resulting loading in the dual loaded beads was 21.4mg/ml rapamycin and 35.5mg/ml doxorubicin.

Assay preparation

The assay was prepared as described in Example 7 using HepG2 cells and additional plates of PSN1 cells for analysis.

Cell viability

Pre-loaded doxorubicin beads, pre-loaded rapamycin beads and dual rapamycin and doxorubicin pre-loaded beads were counted (1, 2 or 3) and added to the prepared 20,000 PSN1 or HepG2 cells in cell culture media and placed in an incubator (37ºC, 5% $CO_2$).

An MTS assay (described in example 7) was conducted after 24, 48 and 72hrs exposure.

The dual loaded bead combination showed greater cell death than either mono-loaded bead at all time points for PSN1 cells (figure 20-22) and HepG2 cells (figure 23-25).

**Example 10: Dual loaded bead compared to two mono-loaded beads**

An MTS assay was used to demonstrate that there was no difference in the cytotoxic effects of one doxorubicin and rapamycin dual-loaded bead compared to two mono-loaded beads (one doxorubicin loaded and one rapamycin loaded).

The drug loaded beads used were the same batches as those described in Example 9.

The assay was setup as described in Example 7 using HepG2 cells. One to four dual loaded beads were added to the prepared 20,000 HepG2 cells in 200μl media and the equivalent dose using mono-loaded beads were added to another well in the same volume of media. The plates were placed in an incubator (37ºC, 5% $CO_2$).

An MTS assay (described in example 7) was conducted after 24, 48 and 72hrs exposure.

The cytotoxic effect of the drug eluting beads was similar regardless of whether the drugs were loaded in one bead or two separate beads (figure 26-28). added to the prepared 20,000 PSN1 or HepG2 cells in cell culture media and placed in an incubator (37°C, 5% $CO_2$).

An MTS assay (described in example 7) was conducted after 24, 48 and 72hrs exposure.

The dual loaded bead combination showed greater cell death than either mono-loaded bead at all time points for PSN1 cells (figure 20-22) and HepG2 cells (figure 23-25).

**Example 10: Dual loaded bead compared to two mono-loaded beads**

An MTS assay was used to demonstrate that there was no difference in the cytotoxic effects of one doxorubicin and rapamycin dual-loaded bead compared to two mono-loaded beads (one doxorubicin loaded and one rapamycin loaded).

The drug loaded beads used were the same batches as those described in Example 9.

The assay was setup as described in Example 7 using HepG2 cells. One to four dual loaded beads were added to the prepared 20,000 HepG2 cells in 200μl media and the equivalent dose using mono-loaded beads were added to another well in the same volume of media. The plates were placed in an incubator (37°C, 5% $CO_2$).

An MTS assay (described in example 7) was conducted after 24, 48 and 72hrs exposure.

The cytotoxic effect of the drug eluting beads was similar regardless of whether the drugs were loaded in one bead or two separate beads (figure 26-28).

## Claims

1. A composition comprising a population of microspheres each of which has a polymer matrix and, jointly incorporated into the matrix, a first and a second pharmaceutically active compound, wherein the polymer is a water insoluble water-swellable polymer which is anionically charged,
   wherein the first active is cationically charged, and the second active is electrostatically neutral and
   wherein one of the actives is a cytotoxic compound and the other active has activity complementary to the cytotoxic compound in tumour treatment.

2. A composition according to claim 1, wherein the first cationically charged compound is a cytotoxic agent.

3. A composition according to claim 1, in which the second active is water-soluble to a degree of less than 10 g/l at room temperature.

4. A composition according to any preceding claim, in which the cytotoxic agent is mitoxantrone or an anthracycline compound having an amine group, of the general formula IV:

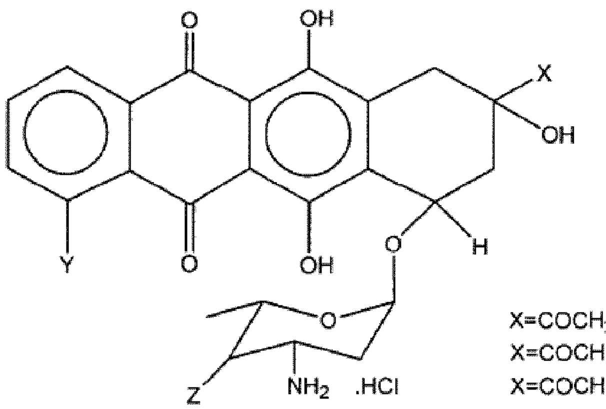

IV

X=$COCH_2OH$ Y=$OCH_3$ Z=HO (axial) Doxorubicin
X=$COCH_3$ Y=$OCH_3$ Z=HO (axial) Daunorubicin
X=$COCH_3$ Y=H Z=HO (axial) Idarubicin
X=$COCH_2OH$ Y=$OCH_3$ Z=HO (equatorial) Epriubicin

5. A composition according to claim 4, in which the anthracycline is doxorubicin.

6. A composition according to any preceding claim, in which the cationic active is a compound of general formula I

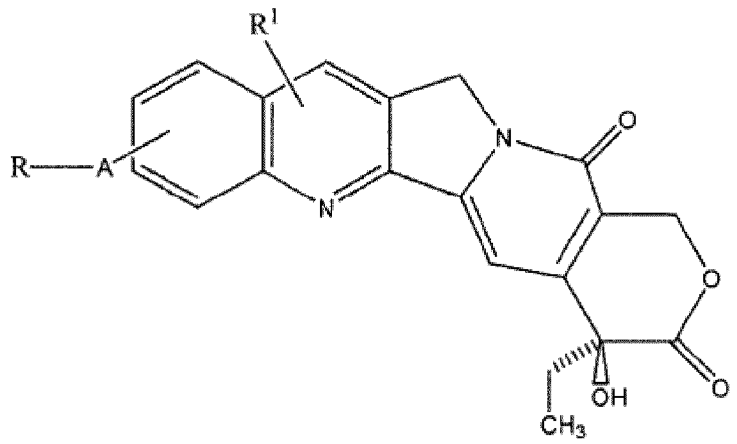

I

in which $R^1$ is selected from H, halogen, hydroxyl and lower ($C_{1-6}$) alkyl, optionally substituted by a hydroxyl, amine, alkoxy, halogen, acyl and acyloxy groups;
A is C(O)O or $CH_2$; and
R is $NR^2R^3$ where $R^2$ and $R^3$ are the same or different and each represents a hydrogen atom, a substituted or unsubstituted $C_{1-4}$ alkyl group or a substituted or unsubstituted carbocyclic or heterocyclic group, or $R^2$ and $R^3$ together with the nitrogen atom to which they are attached form a optionally substituted heterocyclic ring which may be interrupted by -O-, -S- or >$NR^4$ in which $R^4$ is a hydrogen atom, a substituted or unsubstituted $C_{1-4}$ alkyl group or a substituted or unsubstituted phenyl group; and wherein the grouping -A-R is bonded to a carbon atom located in any of the 9, 10 or 11 positions in the A ring of the camptothecin compound and $R^1$ is substituted in the A or B ring, including salts thereof.

7. A composition according to claim 6, in which the cationic active is irinotecan.

8. A compound according to claim 3, in which the low water-solubility compound is rapamycin or a rapamycin analogue, preferably rapamycin.

9. A composition according to any preceding claim, in which the or each polymer is non-biodegradable.

10. A composition according to any preceding claim, in which the or each polymer is water-swellable to an equilibrium water content when swollen in water at 37°C, in the range 40 to 99%, preferably 75 to 95%.

11. A composition according to any preceding claim, in which the or each polymer is wholly synthetic.

12. A composition according to claim 11, in which the or each polymer is crosslinked polyvinyl alcohol.

13. A composition according to claim 12, wherein the polymer matrix is formed from a polyvinyl alcohol macromer, having more than one ethylenically unsaturated pendant group per molecule, by radical polymerisation of the ethylenic groups.

14. A composition according to claim 13, the macromer is copolymerised with ethylenically unsaturated monomers, optionally including a nonionic and/or ionic monomer including anionic monomer.

15. A composition according to claim 14, in which the ethylenically unsaturated monomers include ionic monomer having the general formula III

$$Y^1BQ^1 \quad \text{III}$$

in which $Y^1$ is selected from

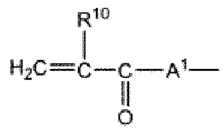 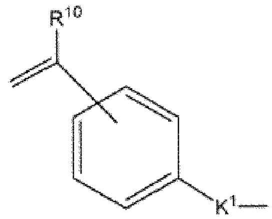

$CH_2=C(R^{10})-CH_2-O-$, $CH_2=C(R^{10})-CH_2OC(O)-$, $CH_2=C(R^{10})OC(O)-$, $CH_2=C(R^{10})-O-$, $CH_2=C(R^{10})CH_2OC(O)N(R^{11})$, $R^{12}OOCCR^{10}=CR^{10}C(O)-O-$, $R^{10}CH=CHC(O)O-$, $R^{10}CH=C(COOR^{12})CH_2-C(O)-O-$,

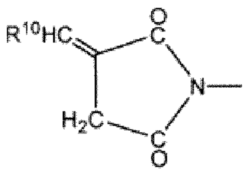 and 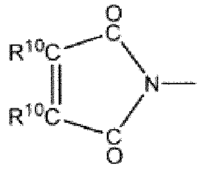

wherein:

$R^{10}$ is hydrogen or a $C_1$-$C_4$ alkyl group;
$R^{11}$ is hydrogen or a $C_1$-$C_4$ alkyl group;
$R^{12}$ is hydrogen or a $C_{1-4}$ alkyl group or $BQ^1$ where B and $Q^1$ are as defined below;
$A^1$ is -O- or $-NR^{11}-$;
$K^1$ is a group $-(CH_2)_rOC(O)-$, $-(CH_2)_rC(O)O-$, $-(CH_2)_rOC(O)O-$, $-(CH_2)_rNR^{13}$, $-(CH_2)_rNR^{13}C(O)-$, $-(CH_2)_rC(O)NR^{13}-$, $-(CH_2)_rNR^{13}C(O)O-$, $-(CH_2)_rOC(O)NR^{13}-$, $-(CH_2)_rNR^{13}C(O)NR^{13}-$ (in which the groups $R^{13}$ are the same or different), $-(CH_2)_rO-$, $-(CH_2)_rS03-$, or, optionally in combination with B, a valence bond and r is from 1 to 12 and $R^{13}$ is hydrogen or a $C_1$-$C_4$ alkyl group;
B is a straight or branched alkanediyl, oxaalkylene, alkanediyloxaalkanediyl, or alkanediyloligo(oxaalkanediyl) chain optionally containing one or more fluorine atoms up to and including perfluorinated chains or, if $Q^1$ or $Y^1$ contains a terminal carbon atom bonded to B a valence bond; and
$Q^1$ is an anionic group.

**16.** A composition according to claim 15, in which $Q^1$ is an anionic group, preferably a carboxylate, carbonate, sulphonate, sulphate, nitrate, phosphonate or phosphate group.

**17.** A method of loading two actives jointly into microspheres involving the steps:

contacting microspheres which comprise a matrix of a water-insoluble water-swellable anionically charged crosslinked polymer in substantially dry form with a solution of a drug having a water-solubility less than 10 g/l at room temperature in a first organic solvent, whereby a solution of drug in solvent becomes impregnated into the microspheres;
separating drug solution which has not impregnated the microspheres;
contacting the impregnated microspheres with aqueous liquid whereby drug is precipitated in the core of the microspheres;
contacting the microspheres containing precipitated drug with an aqueous solution of an ionic drug having a charge opposite to that of the polymer to allow loading of ionexchange; and recovering the dual loaded microspheres, wherein the drugs are selected as in any of claims 1 to 10.

**18.** A method of loading two pharmaceutical actives jointly into a drug delivery matrix comprising the steps:

a drug delivery matrix which comprises a water-insoluble, water-swellable ionically charged polymer which is swellable in water at room temperature to an equilibrium water content in the range 40 to 99% by weight based on polymer plus water is contacted with a solution of drug having a water-solubility less than 10g/l at room temperature in an organic solvent which is capable of swelling the polymer, whereby the solution becomes impregnated into the matrix;
any drug solution which has not impregnated the polymer matrix is separated;

the impregnated matrix is contacted with water whereby drug is precipitated in the matrix;
the matrix having precipitated drug having low water solubility is contacted with an aqueous solution of an ionically charged drug whereby the matrix swells and ionexchange with counter ions associated with the polymer takes place and charged drug is electrostatically bound in the polymer matrix.

**19.** . A method according to claim 17 or 18, in which the active having a water-solubility of less than 10 g/l is rapamycin or an analogue.

**20.** . A method according to claim 18, in which the ionically charged active is a mitoxantrone or anthracycline compound having an amine group, preferably doxorubicin.

**21.** . A composition according to any of claims 1 to 16, for use in a method of treating a solid tumour by administration to an animal suffering from a tumour in such a manner that the first and second actives are each delivered at the target tumour.

**22.** . A composition for use in a method according to claim 21, for intratumoural injection, or for injection at the resection margin after removal of the tumour by surgery or where the matrix is in the form of microspheres, having diameters in the range 100 to 1500 um when swollen in distilled water at 37°C, for embolisation of a blood vessel feeding the tumour.

**23.** . A composition for use in a method according to claim 22, in which the tumour is a hepatic tumour.

**Patentansprüche**

**1.** Zusammensetzung, umfassend eine Gesamtheit von Mikrokugeln, von denen jede eine Polymermatrix und, gemeinsam in die Matrix eingebaut, eine erste und eine zweite pharmazeutisch wirksame Verbindung aufweist, wobei das Polymer ein wasserunlösliches, in Wasser quellbares Polymer ist, das anionisch geladen ist,
wobei der erste Wirkstoff kationisch geladen ist und der zweite Wirkstoff elektrostatisch neutral ist und
wobei einer der Wirkstoffe eine zytotoxische Verbindung ist und der andere Wirkstoff eine Wirksamkeit aufweist, die zur zytotoxischen Verbindung bei der Tumorbehandlung komplementär ist.

**2.** Zusammensetzung nach Anspruch 1, wobei die erste kationisch geladene Verbindung ein zytotoxisches Mittel ist.

**3.** Zusammensetzung nach Anspruch 1, wobei der zweite Wirkstoff bis zu einem Maß von weniger als 10 g/l bei Raumtemperatur wasserlöslich ist.

**4.** Zusammensetzung nach einem der vorstehenden Ansprüche, wobei der zytotoxische Wirkstoff Mitoxantron oder eine Anthracyclin-Verbindung, die eine Amingruppe aufweist, der allgemeinen Formel IV ist:

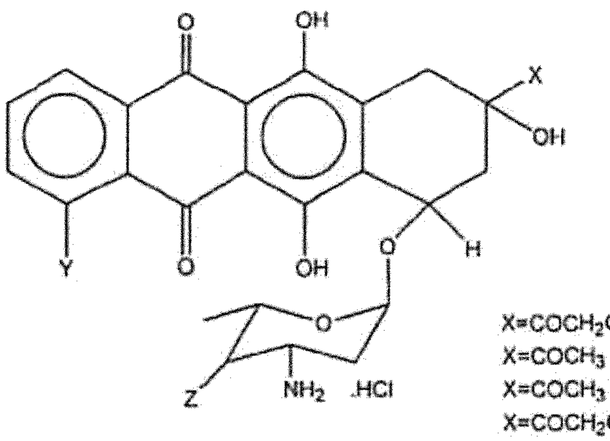

IV

$X=COCH_2OH$ $Y=OCH_3$ Z=HO (axial) Doxorubicin
$X=COCH_3$ $Y=OCH_3$ Z=HO (axial) Daunorubicin
$X=COCH_3$ Y=H Z=HO (axial) Idarubicin
$X=COCH_2OH$ $Y=OCH_3$ Z=HO (äquatorial ) Epriubicin

**5.** Zusammensetzung nach Anspruch 4, wobei das Anthracyclin Doxorubicin ist.

**6.** Zusammensetzung nach einem der vorstehenden Ansprüche, wobei der kationische Wirkstoff eine Verbindung der

allgemeinen Formel I ist

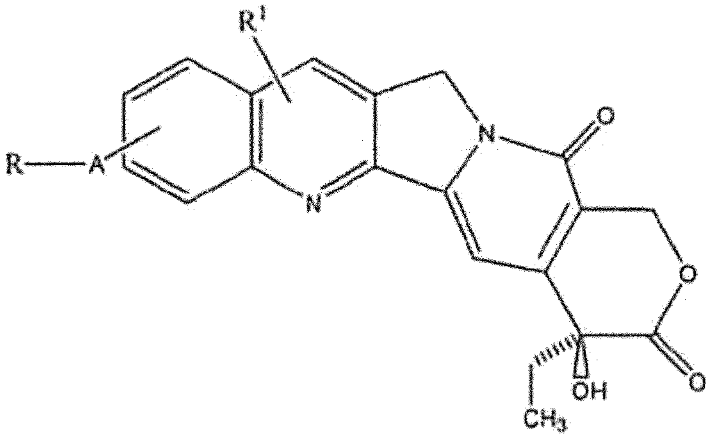

I

wobei $R^1$ ausgewählt ist aus H, Halogen, Hydroxyl- und Nieder-($C_{1-6}$) Alkyl, optional substituiert durch eine Hydroxyl-, Amin-, Alkoxy-, Halogen-, Acyl- und Acyloxy-Gruppen;
A C(O)O oder $CH_2$ ist; und
R $NR^2R^3$ ist, wobei $R^2$ und $R^3$ gleich oder unterschiedlich sind und jeweils ein Wasserstoffatom, eine substituierte oder nicht substituierte $C_{1-4}$-Alkylgruppe oder eine substituierte oder nicht substituierte carbocyclische oder heterocyclische Gruppe darstellen, oder $R^2$ und $R^3$ gemeinsam mit den Stickstoffatom, an dem sie befestigt sind, einen optional substituierten heterocyclischen Ring bilden, der durch -O-, -S- oder >$NR^4$ unterbrochen sein kann, wobei $R^4$ ein Wasserstoffatom, eine substituierte oder nicht substituierte $C_{1-4}$-Alkylgruppe oder eine substituierte oder nicht substituierte Phenylgruppe ist; und wobei die Gruppierung -A-R an ein Kohlenstoffatom gebunden ist, das an einer der Positionen 9, 10 oder 11 im A-Ring der Camptothecin-Verbindung angeordnet ist, und $R^1$ im A- oder B-Ring substituiert ist, einschließlich deren Salze.

**7.** Zusammensetzung nach Anspruch 6, wobei der kationische Wirkstoff Irinotecan ist.

**8.** Zusammensetzung nach Anspruch 3, wobei die Verbindung mit geringer Wasserlöslichkeit Rapamycin oder eine Rapamycin-Analogon, vorzugsweise Rapamycin, ist.

**9.** Zusammensetzung nach einem der vorstehenden Ansprüche, wobei das oder jedes Polymer nicht biologisch abbaubar ist.

**10.** Zusammensetzung nach einem der vorstehenden Ansprüche, wobei das oder jedes Polymer bis zu einem Gleichgewichtswassergehalt nach Quellen in Wasser bei 37 °C im Bereich von 40 bis 99 %, vorzugsweise von 75 bis 95 % in Wasser quellbar ist.

**11.** Zusammensetzung nach einem der vorstehenden Ansprüche, wobei das oder jedes Polymer vollständig synthetisch ist.

**12.** Zusammensetzung nach Anspruch 11, wobei das oder jedes Polymer vernetzter Polyvinylalkohol ist.

**13.** Zusammensetzung nach Anspruch 12, wobei die Polymermatrix aus einem Polyvinylalkohol-Makromer, das mehr als eine ethylenisch ungesättigte Seitengruppe je Molekül aufweist, durch radikalische Polymerisation der ethylenischen Gruppen gebildet wird.

**14.** Zusammensetzung nach Anspruch 13, wobei das Makromer mit ethylenisch ungesättigten Monomeren copoylmerisiert ist, optional umfassend ein nichtionisches und/oder ionisches Monomer einschließlich eines anionischen Monomers.

**15.** Zusammensetzung nach Anspruch 14, wobei die ethylenisch ungesättigten Monomere ionisches Monomer enthalten, das die allgemeine Formel III aufweist

$$Y^1BQ^1 \qquad III$$

wobei $Y^1$ ausgewählt ist aus

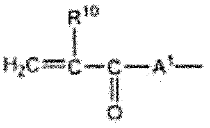

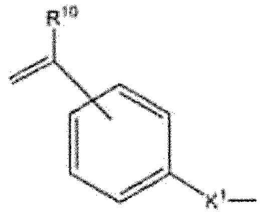

$CH_2=XR^{10})-CH_2-O-$, $CH_2=C(R^{10})-CH_2$ $OC(O)-$, $CH_2=C(R^{10})OC(O)-$, $CH_2=C(R^{10})$. $O-$, $CH_2=C(R^{10})CH_2OC(O)N(R^{11})-$, $R^{12}OOCCR^{10}=CR^{10}C(O)-O-$, $R^{10}CH=CHC(O)O-$, $R^{10}CH=C(COOR^{12})CH_2-C(O)-O-$,

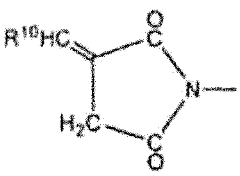

und

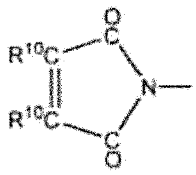

wobei:

$R^{10}$ Wasserstoff oder eine $C_1$-$C_4$-Alkylgruppe ist;
$R^{11}$ Wasserstoff oder eine $C_1$-$C_4$-Alkylgruppe ist;
$R^{12}$ Wasserstoff oder eine $C_{1-4}$-Alkylgruppe oder $BQ^1$ ist, wobei B und $Q^1$ wie nachstehend definiert sind;
$A^1$ -O- oder $-NR^{11}-$ ist;
$K^1$ eine Gruppe $-(CH_2)_rOC(O)-$, $-(CH_2)_rC(O)O-$, $-(CH_2)_rOC(O)O-$,$-(CH_2)_rNR^{13}$,$-(CH_2)_rNR^{13}C(O)-$, $-(CH_2)_rC(O)NR^{13}-$, $-(CH_2)_rNR^{13}C(O)O-$,$-(CH_2)_rOC(O)NR^{13}-$, $-(CH_2)_rNR^{13}C(O)NR^{13}-$ (wobei die Gruppen $R^{13}$ identisch oder verschieden sind), $-(CH_2)_rO-$, $-(CH_2)_rSO3-$ ist oder optional in Kombination mit B, eine Valenzbindung ist und r von 1 bis 12 ist und $R^{13}$ Wasserstoff oder eine $C_1$-$C_4$-Alkylgruppe ist; B eine unverzweigte oder verzweigte Alkandiyl-, Oxaalkylen-, Alkandiyloxaalkandiyl- oder Alkandiyloligo(oxaalkandiyl)-Kette ist, optional enthaltend ein oder mehrere Fluoratome bis zu einschließlich perfluorierten Ketten oder, wenn $Q^1$ oder $Y^1$ ein terminales Kohlenstoffatom, das an B gebunden ist, enthält, eine Valenzbindung ist; und
$Q^1$ eine anionische Gruppe ist.

**16.** Zusammensetzung nach Anspruch 15, wobei $Q^1$ eine anionische Gruppe, vorzugsweise eine Carboxylat-, Carbonat-, Sulfonat-, Sulfat-, Nitrat-, Phosphonat- oder Phosphatgruppe ist.

**17.** Verfahren zum gemeinsamen Laden zweier Wirkstoffe in Mikrokugeln, das die folgenden Schritte umfasst:

Herstellen des Kontakts zwischen Mikrokugeln, die eine Matrix aus einem wasserunlöslichen, in Wasser quellbaren, anionisch geladenen, vernetzten Polymer in im Wesentlichen trockener Form und einer Lösung eines Arzneimittels mit einer Wasserlöslichkeit von weniger als 10 g/l bei Raumtemperatur in einem ersten organischen Lösungsmittel, wobei die Mikrokugeln mit einer Lösung des Arzneimittels im Lösungsmittel durchtränkt werden;
Abtrennen der Arzneimittellösung, die die Mikrokugeln nicht durchtränkt hat;
Herstellen des Kontakts zwischen den durchtränkten Mikrokugeln mit wässriger Flüssigkeit, wobei das Arzneimittel im Kern der Mikrokugeln ausgefällt wird;
Herstellen des Kontakts zwischen den Mikrokugeln, die das ausgefällte Arzneimittel enthalten, mit einer wässrigen Lösung eines ionischen Arzneimittels, das eine Ladung aufweist, die derjenigen des Polymers entgegengesetzt ist, um das Laden mit Ionenaustausch zu ermöglichen; und Wiedergewinnen der doppelt geladenen Mikrokugeln, wobei die Arzneimittel wie in einem der Ansprüche 1 bis 10 ausgewählt sind.

**18.** Verfahren zum gemeinsamen Laden zweier pharmazeutischer Wirkstoffe in eine Arzneimittelabgabematrix, umfassend die Schritte:

eine Arzneimittelabgabematrix, die ein wasserunlösliches, in Wasser quellbares, ionisch geladenes Polymer

umfasst, das in Wasser bei Raumtemperatur bis zu einem Gleichgewichtswassergehalt im Bereich von 40 bis 99 Gew.-% basierend auf Polymer plus Wasser quellbar ist, wird mit einer Lösung eines Arzneimittels mit einer Wasserlöslichkeit von weniger als 10 g/l bei Raumtemperatur in einem organischen Lösungsmittel, das in der Lage ist, das Polymer zum Quellen zu bringen, in Kontakt gebracht, wobei die Lösung die Matrix durchtränkt;
jegliche Arzneimittellösung, die die Polymermatrix nicht durchtränkt hat, wird abgetrennt;
die getränkte Matrix wird mit Wasser in Kontakt gebracht, wodurch das Arzneimittel in der Matrix ausgefällt wird;
die Matrix wird nach dem Ausfällen des Arzneimittels mit niedriger Wasserlöslichkeit mit einer wässrigen Lösung eines ionisch geladenen Arzneimittels in Kontakt gebracht, wodurch die Matrix quillt und ein Ionenaustausch mit Gegenionen, die zum Polymer gehören, stattfindet und das geladene Arzneimittel elektrostatisch in die Polymermatrix gebunden wird.

**19.** Verfahren nach Anspruch 17 oder 18, wobei der Wirkstoff mit einer Wasserlöslichkeit von weniger als 10 g/l Rapamycin oder ein Analogon ist.

**20.** Verfahren nach Anspruch 18, wobei der ionisch geladene Wirkstoffeine Mitoxantron- oder eine Anthracyclin-Verbindung ist, die eine Amingruppe aufweist, vorzugsweise Doxorubicin.

**21.** Zusammensetzung nach einem der Ansprüche 1 bis 16 zur Verwendung in einem Verfahren zur Behandlung eines soliden Tumors durch Verabreichung an ein Tier, das an einem Tumor leidet, in einer Weise, so dass der erste und zweite Wirkstoff jeweils am Zieltumor abgegeben werden.

**22.** Zusammensetzung zur Verwendung in einem Verfahren nach Anspruch 21 zur intratumoralen Injektion oder zur Injektion am Resektionsrand nach Entfernung des Tumors durch Operation oder, wenn die Matrix in Form von Mikrokugeln vorliegt, die Durchmesser im Bereich von 100 bis 1.500 um aufweisen, wenn sie in destilliertem Wasser bei 37 °C gequollen sind, zur Embolisation von Blutgefäßen, die den Tumor versorgen.

**23.** Zusammensetzung zur Verwendung in einem Verfahren nach Anspruch 22, wobei der Tumor ein Lebertumor ist.

## Revendications

**1.** Composition comprenant une population de microsphères dont chacune a une matrice polymère et, incorporés conjointement dans la matrice, un premier et un second composé pharmaceutiquement actif, dans laquelle le polymère est un polymère gonflable dans l'eau, insoluble dans l'eau qui est anioniquement chargé,
dans laquelle le premier actif est cationiquement chargé, et le second actif est électrostatiquement neutre et
dans laquelle l'un des actifs est un composé cytotoxique et l'autre actif a une activité complémentaire au composé cytotoxique dans le traitement des tumeurs.

**2.** Composition selon la revendication 1, dans laquelle le premier composé cationiquement chargé est un agent cytotoxique.

**3.** Composition selon la revendication 1, dans laquelle le second actif est soluble dans l'eau à un degré inférieur à 10 g/l à température ambiante.

**4.** Composition selon une quelconque revendication précédente, dans laquelle l'agent cytotoxique est la mitoxantrone ou un composé anthracycline ayant un groupe amine, de formule générale IV :

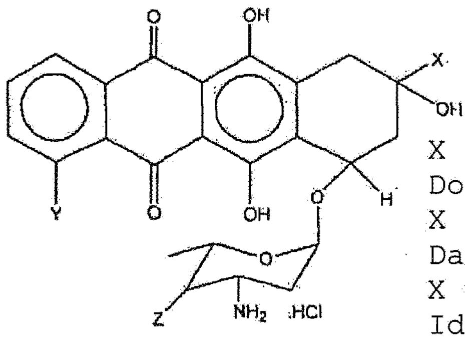

IV

X = $COCH_2OH$ Y = $OCH_3$ Z = HO (axiale) Doxorubicine

X = $COCH_3$ Y = $OCH_3$ Z = HO (axiale) Daunorubicine

X = $COCH_3$ Y = H Z = HO (axiale ) Idarubicine

X = $COCH_2OH$ Y = $OCH_3$ Z = HO (équatoriale) Épirubicine

**5.** Composition selon la revendication 4, dans laquelle l'anthracycline est la doxorubicine.

**6.** Composition selon une quelconque revendication précédente, dans laquelle l'actif cationique est un composé de formule générale I

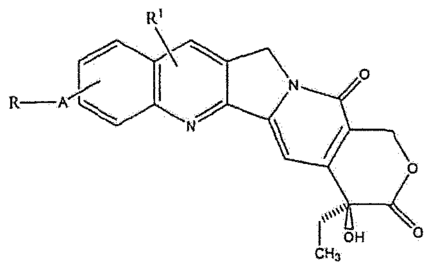

1

dans laquelle $R^1$ est choisi parmi un H, un halogène, un hydroxyle et un alkyle ($C_{1-6}$) inférieur, éventuellement substitué par un hydroxyle, une amine, un alcoxy, un halogène, un acyle et des groupes acyloxy ;
A représente C(O)O ou $CH_2$ ; et
R représente $NR^2R^3$ où $R^2$ et $R^3$ sont identiques ou différents et représentent chacun un atome d'hydrogène, un groupe alkyle en $C_{1-4}$ substitué ou non substitué ou un groupe carbocyclique ou hétérocyclique substitué ou non substitué, ou $R^2$ et $R^3$ conjointement avec l'atome d'azote auquel ils sont liés à partir d'un cycle hétérocyclique éventuellement substitué qui peut être interrompu par -O-, -S- ou >$NR^4$ dans lequel $R^4$ est un atome d'hydrogène, un groupe alkyle en $C_{1-4}$ substitué ou non substitué ou un groupe phényle substitué ou non substitué ; et dans lequel le groupement -A-R est lié à un atome de carbone situé dans l'une quelconque des positions 9, 10 ou 11 dans le cycle A du composé camptothécine et $R^1$ est substitué dans le cycle A ou B, comprenant les sels de celui-ci.

**7.** Composition selon la revendication 6, dans laquelle l'actif cationique est l'irinotécan.

**8.** Composé selon la revendication 3, dans lequel le composé à faible solubilité dans l'eau est la rapamycine ou un analogue de la rapamycine, de préférence la rapamycine.

**9.** Composition selon une quelconque revendication précédente, dans laquelle le ou chaque polymère est non biodégradable.

**10.** Composition selon une quelconque revendication précédente, dans laquelle le ou chaque polymère est gonflable dans l'eau jusqu'à une teneur en eau d'équilibre lorsqu'il est gonflé dans l'eau à 37 °C, dans la plage de 40 à 99 %, de préférence de 75 à 95 %.

**11.** Composition selon une quelconque revendication précédente, dans laquelle le ou chaque polymère est entièrement synthétique.

**12.** Composition selon la revendication 11, dans laquelle le ou chaque polymère est un alcool polyvinylique réticulé.

**13.** Composition selon la revendication 12, dans laquelle la matrice polymère est formée à partir d'un macromère d'alcool polyvinylique, ayant plus d'un groupe latéral éthyléniquement insaturé par molécule, par polymérisation radicalaire des groupes éthyléniques.

**14.** Composition selon la revendication 13, le macromère est copolymérisé avec des monomères éthyléniquement insaturés, comprenant éventuellement un monomère non ionique et/ou ionique comprenant un monomère anionique.

**15.** Composition selon la revendication 14, dans laquelle les monomères éthyléniquement insaturés comprennent un monomère ionique répondant à la formule générale III.

$Y^1BQ^1$ III

dans laquelle $Y^1$ est choisi parmi

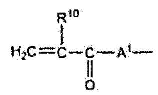 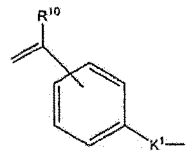

$CH_2{=}C(R^{10})\text{-}CH_2\text{-}O\text{-}$, $CH_2{=}C(R^{10})\text{-}CH_2OC(O)\text{-}$, $CH_2{=}C(R^{10})OC(O)\text{-}$, $CH_2{=}C(R^{10})\text{-}O\text{-}$, $CH_2{=}C(R^{10})CH_2OC(O)N(R^{11})\text{-}$, $R^{12}OOCCR^{10}{=}CR^{10}C(O)\text{-}O\text{-}$, $R^{10}CH{=}CHC(O)O\text{-}$, $R^{10}CH{=}C(COOR^{12})CH_2\text{-}C(O)\text{-}O\text{-}$,

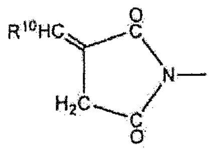 et 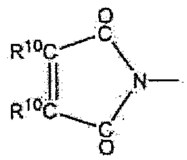

dans laquelle :

$R^{10}$ représente un hydrogène ou un groupe alkyle en $C_1$-$C_4$ ;
$R^{11}$ représente un hydrogène ou un groupe alkyle en $C_1$-$C_4$ ;
$R^{12}$ représente un hydrogène ou un groupe alkyle en $C_{1-4}$ ou $BQ^1$ où B et $Q^1$ sont tels que définis ci-dessous ;
$A^1$ représente -O- ou $-NR^{11}-$ ;
$K^1$ représente un groupe $-(CH_2)_rOC(O)-$, $-(CH_2)_rC(O)O-$,$-(CH_2)_rOC(O)O-$, $-(CH_2)_rNR^{13}$, $-(CH_2)_rNR^{13}C(O)-$, $-(CH_2)_rC(O)NR^{13}-$,$-(CH_2)_rNR^{13}C(O)O-$, $-(CH_2)_rOC(O)NR^{13}-$, - $(CH_2)_rNR^{13}C(O)NR^{13}-$ (dans lesquels les groupes $R^{13}$ sont identiques ou différents),$-(CH_2)_rO-$, $-(CH_2)_rS03-$, ou, éventuellement en combinaison avec B, une liaison de valence et r va de 1 à 12 et $R^{13}$ représente un hydrogène ou un groupe alkyle en $C_1$-$C_4$ ;
B représente une chaîne alcanediyle, oxaalkylène, alcanediyloxaalcanediyle ou alcanediyloligo(oxaalcanediyle) droite ou ramifiée contenant éventuellement un ou plusieurs atomes de fluor jusqu'à et comprenant des chaînes perfluorées ou, si $Q^1$ ou $Y^1$ contient un atome de carbone terminal lié à B, une liaison de valence ; et
$Q^1$ représente un groupe anionique.

**16.** Composition selon la revendication 15, dans laquelle $Q^1$ représente un groupe anionique, de préférence un groupe carboxylate, carbonate, sulfonate, sulfate, nitrate, phosphonate ou phosphate.

**17.** Procédé de chargement de deux actifs conjointement dans des microsphères impliquant les étapes de :

mise en contact des microsphères qui comprennent une matrice d'un polymère réticulé anioniquement chargé gonflable dans l'eau, insoluble dans l'eau sous une forme sensiblement sèche avec une solution d'un médicament d'une solubilité dans l'eau inférieure à 10 g/l à température ambiante dans un premier solvant organique, moyennant quoi une solution de médicament dans le solvant s'imprègne dans les microsphères ;
séparation de la solution de médicament qui n'a pas imprégné les microsphères ;
mise en contact des microsphères imprégnées avec un liquide aqueux, moyennant quoi le médicament est précipité dans le noyau des microsphères ;

mise en contact des microsphères contenant le médicament précipité avec une solution aqueuse d'un médicament ionique ayant une charge opposée à celle du polymère pour permettre le chargement de l'échange d'ions ; et récupération des microsphères à double charge, dans lequel les médicaments sont sélectionnés selon l'une quelconque des revendications 1 à 10.

**18.** Procédé de chargement de deux actifs pharmaceutiques conjointement dans une matrice d'administration de médicament comprenant les étapes :

une matrice d'administration de médicament qui comprend un polymère ioniquement chargé gonflable dans l'eau, insoluble dans l'eau qui est gonflable dans l'eau à température ambiante jusqu'à une teneur en eau d'équilibre dans la plage de 40 à 99 % en poids sur une base polymère plus eau est mise en contact avec une solution de médicament ayant une solubilité dans l'eau inférieure à 10 g/l à température ambiante dans un solvant organique qui est capable de gonfler le polymère, moyennant quoi la solution s'imprègne dans la matrice ;
toute solution de médicament qui n'a pas imprégné la matrice polymère est séparée ;
la matrice imprégnée est mise en contact avec de l'eau, moyennant quoi le médicament est précipité dans la matrice ;
la matrice comportant le médicament précipité ayant une faible solubilité dans l'eau est mise en contact avec une solution aqueuse d'un médicament ioniquement chargé, moyennant quoi la matrice gonfle et un échange d'ions avec des contre-ions associés au polymère a lieu et le médicament chargé est électrostatiquement lié dans la matrice polymère.

**19.** Procédé selon la revendication 17 ou 18, dans lequel l'actif ayant une solubilité dans l'eau inférieure à 10 g/l est la rapamycine ou un analogue.

**20.** Procédé selon la revendication 18, dans lequel l'actif ioniquement chargé est un composé mitoxantrone ou anthracycline ayant un groupe aminé, de préférence la doxorubicine.

**21.** Composition selon l'une quelconque des revendications 1 à 16, destinée à être utilisée dans un procédé de traitement d'une tumeur solide par l'administration à un animal souffrant d'une tumeur de telle manière que les premier et second actifs sont chacun administrés au niveau de la tumeur cible.

**22.** Composition destinée à être utilisée dans un procédé selon la revendication 21, pour une injection intratumorale, ou pour une injection au niveau de la marge de résection après l'ablation de la tumeur par chirurgie ou lorsque la matrice est sous forme de microsphères, ayant des diamètres dans la plage de 100 à 1 500 um lorsqu'elle est gonflée dans de l'eau distillée à 37 °C, pour l'embolisation d'un vaisseau sanguin alimentant la tumeur.

**23.** Composition destinée à être utilisée dans un procédé selon la revendication 22, dans laquelle la tumeur est une tumeur hépatique.

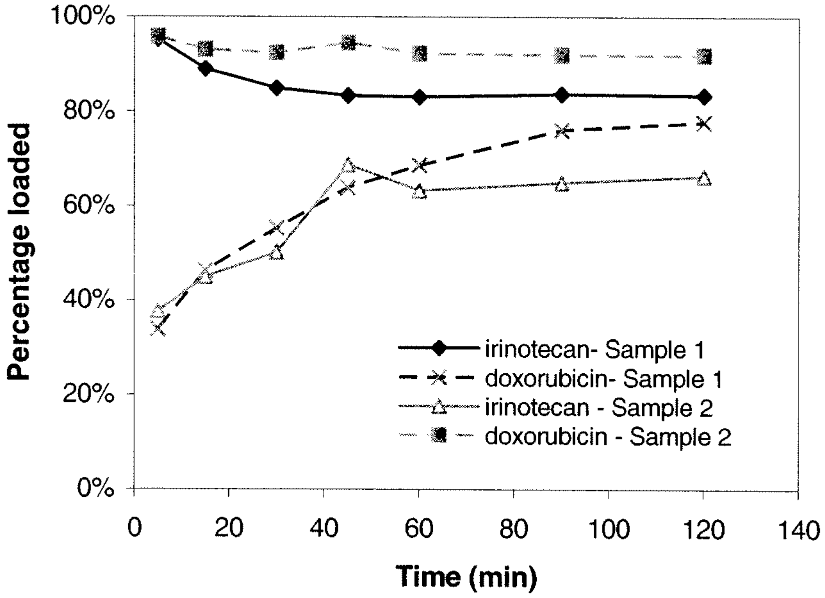


Figure 1

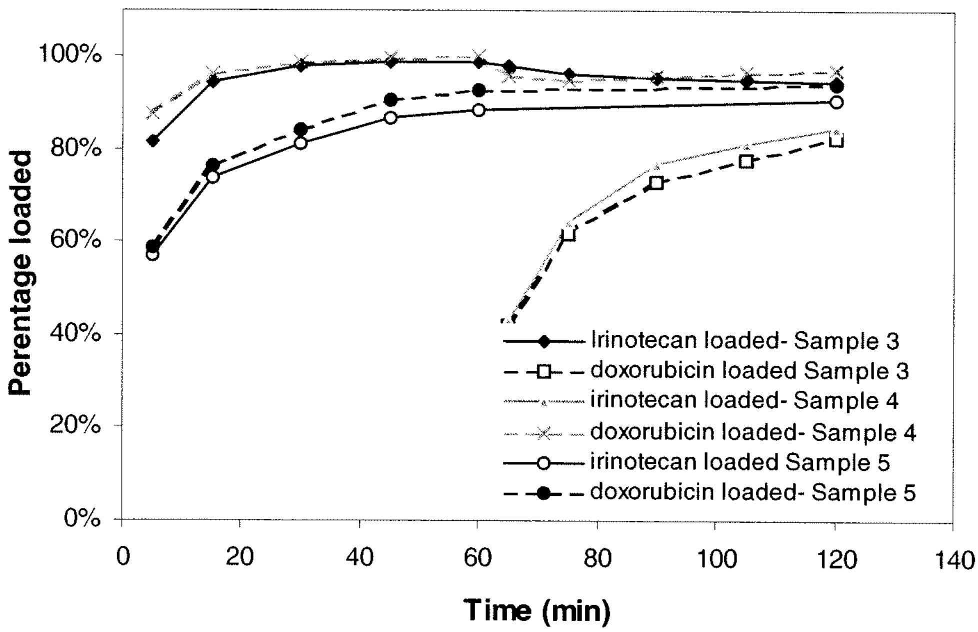


Figure 2

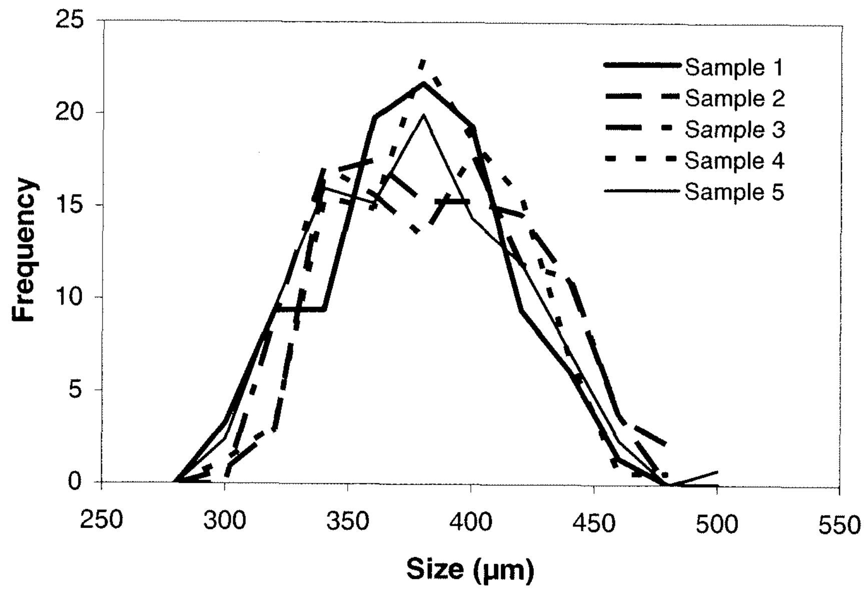


Figure 3.

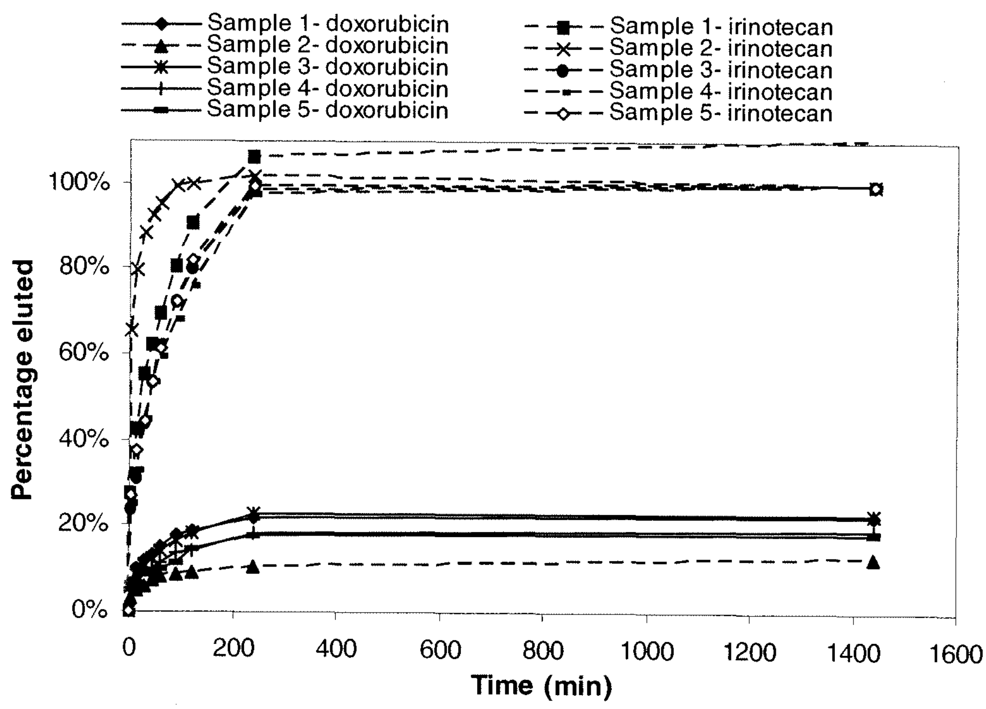


Figure 4

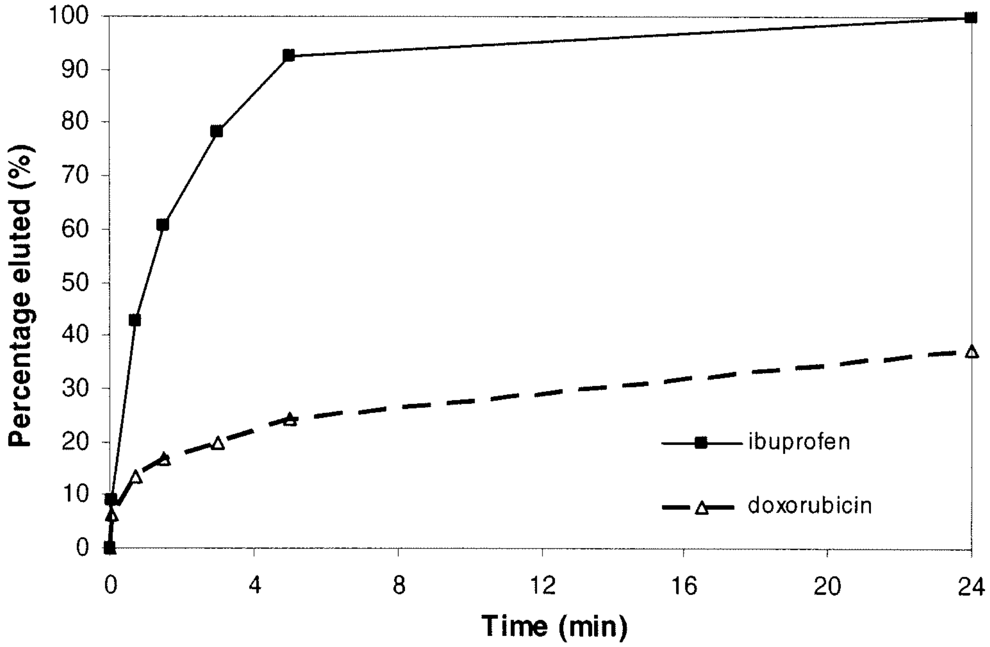
100
90
80
70
60
50
40
30
20
10
0
Percentage eluted (%)
0
4
8
12
16
20
24
Time (min)
ibuprofen
doxorubicin

Figure 5

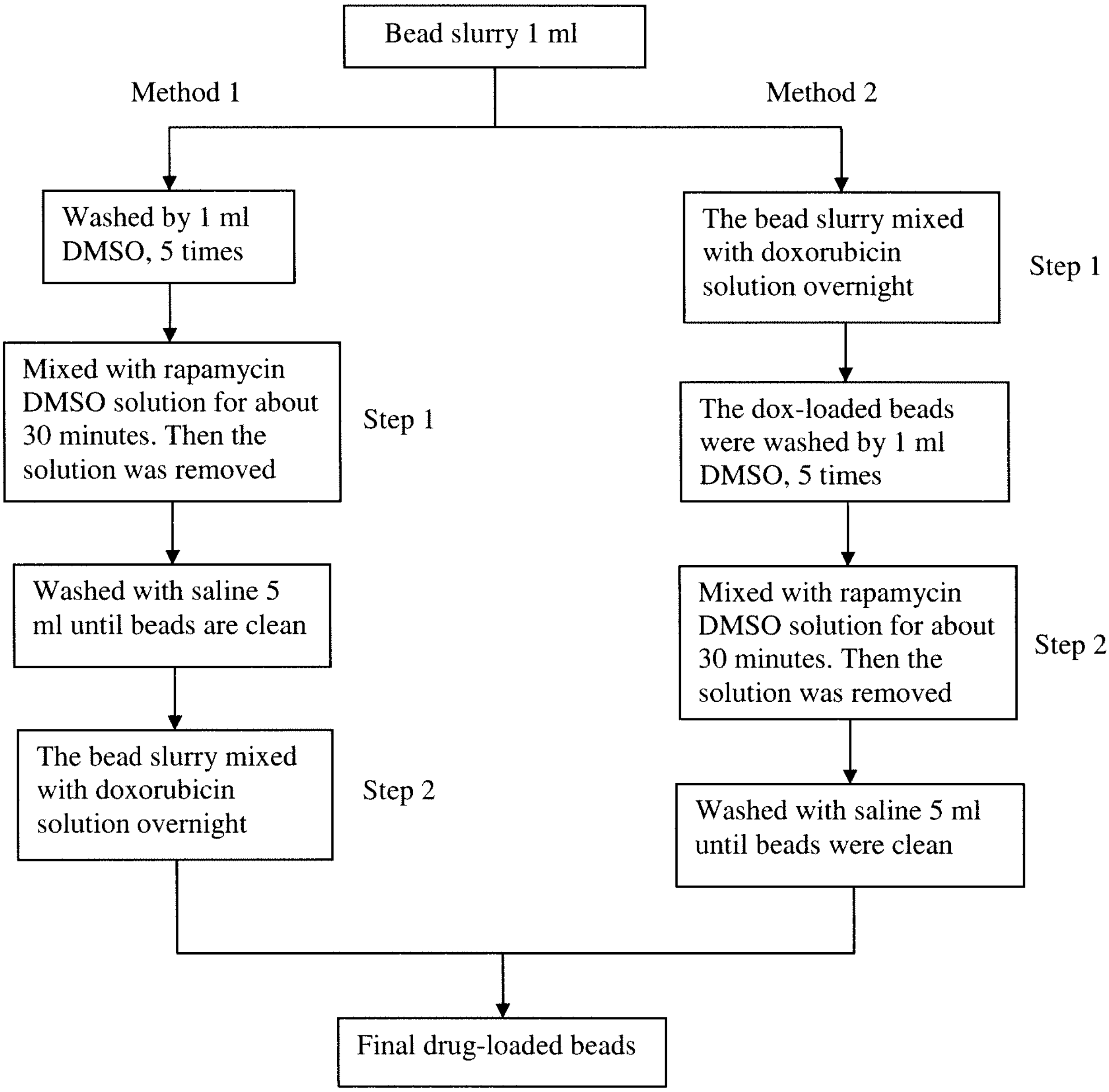
Bead slurry 1 ml
Method 1
Method 2
Washed by 1 ml DMSO, 5 times
Mixed with rapamycin DMSO solution for about 30 minutes. Then the solution was removed
Step 1
Washed with saline 5 ml until beads are clean
The bead slurry mixed with doxorubicin solution overnight
Step 2
The bead slurry mixed with doxorubicin solution overnight
Step 1
The dox-loaded beads were washed by 1 ml DMSO, 5 times
Mixed with rapamycin DMSO solution for about 30 minutes. Then the solution was removed
Step 2
Washed with saline 5 ml until beads were clean
Final drug-loaded beads

**Figure 6**

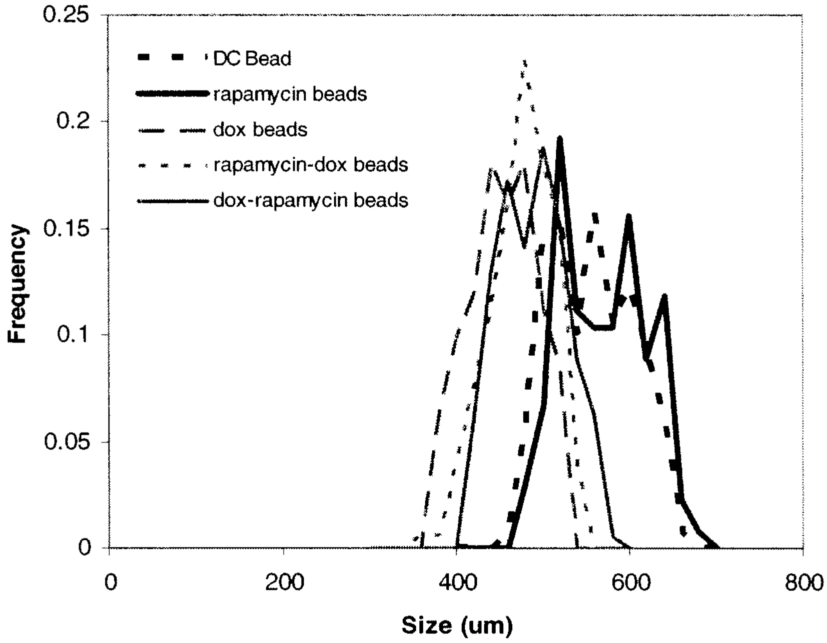

0.25
0.2
0.15
0.1
0.05
0
Frequency
DC Bead
rapamycin beads
dox beads
rapamycin-dox beads
dox-rapamycin beads
0
200
400
600
800
Size (um)


Figure 7

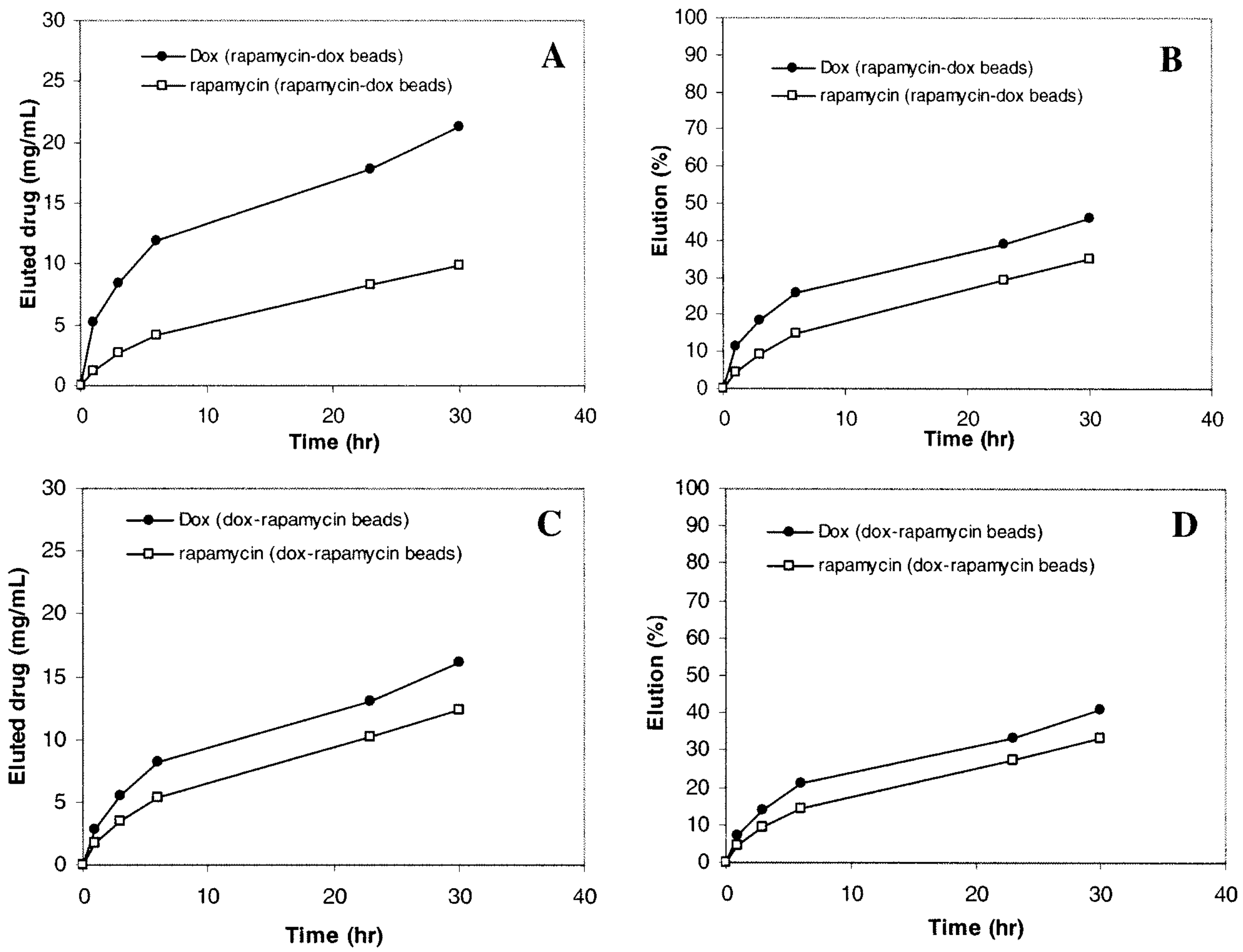
A
Dox (rapamycin-dox beads)
rapamycin (rapamycin-dox beads)
Eluted drug (mg/mL)
Time (hr)
B
Dox (rapamycin-dox beads)
rapamycin (rapamycin-dox beads)
Elution (%)
Time (hr)
C
Dox (dox-rapamycin beads)
rapamycin (dox-rapamycin beads)
Eluted drug (mg/mL)
Time (hr)
D
Dox (dox-rapamycin beads)
rapamycin (dox-rapamycin beads)
Elution (%)
Time (hr)

Figures 8 A-D

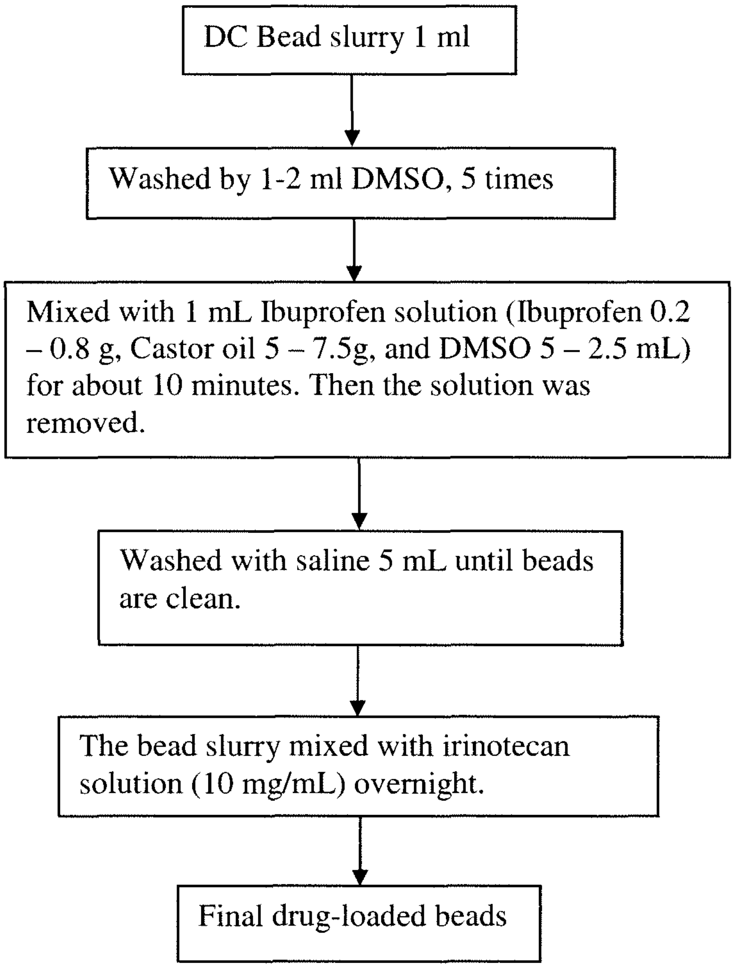
DC Bead slurry 1 ml
Washed by 1-2 ml DMSO, 5 times
Mixed with 1 mL Ibuprofen solution (Ibuprofen 0.2 – 0.8 g, Castor oil 5 – 7.5g, and DMSO 5 – 2.5 mL) for about 10 minutes. Then the solution was removed.
Washed with saline 5 mL until beads are clean.
The bead slurry mixed with irinotecan solution (10 mg/mL) overnight.
Final drug-loaded beads

**Figure 9**

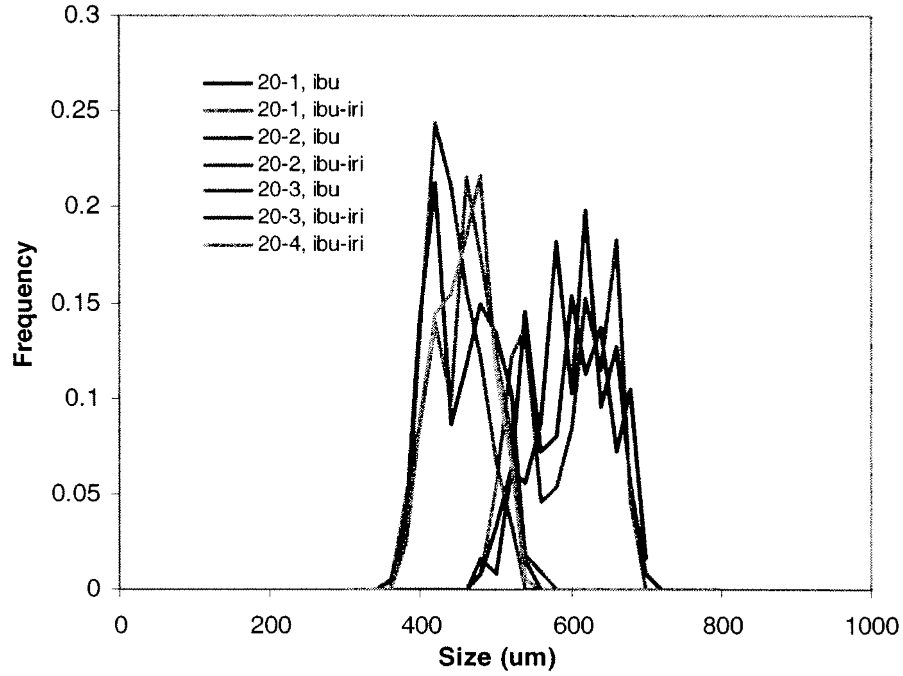


**Figure 10**

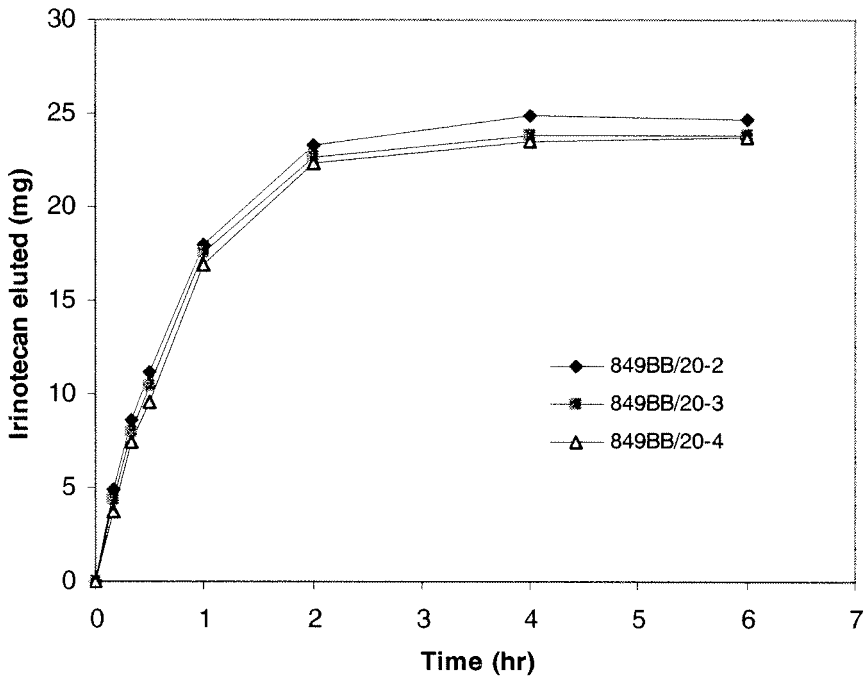


**Figure 11**

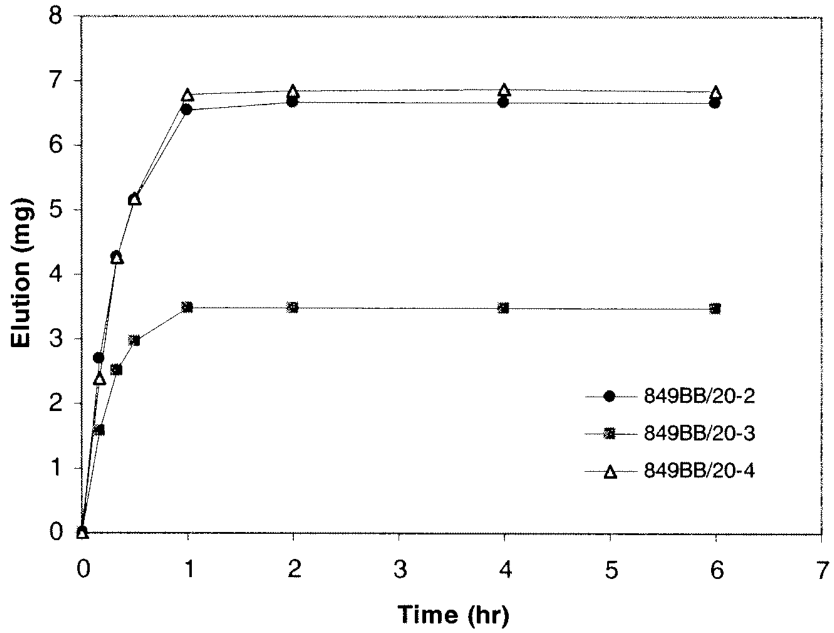


Figure 12

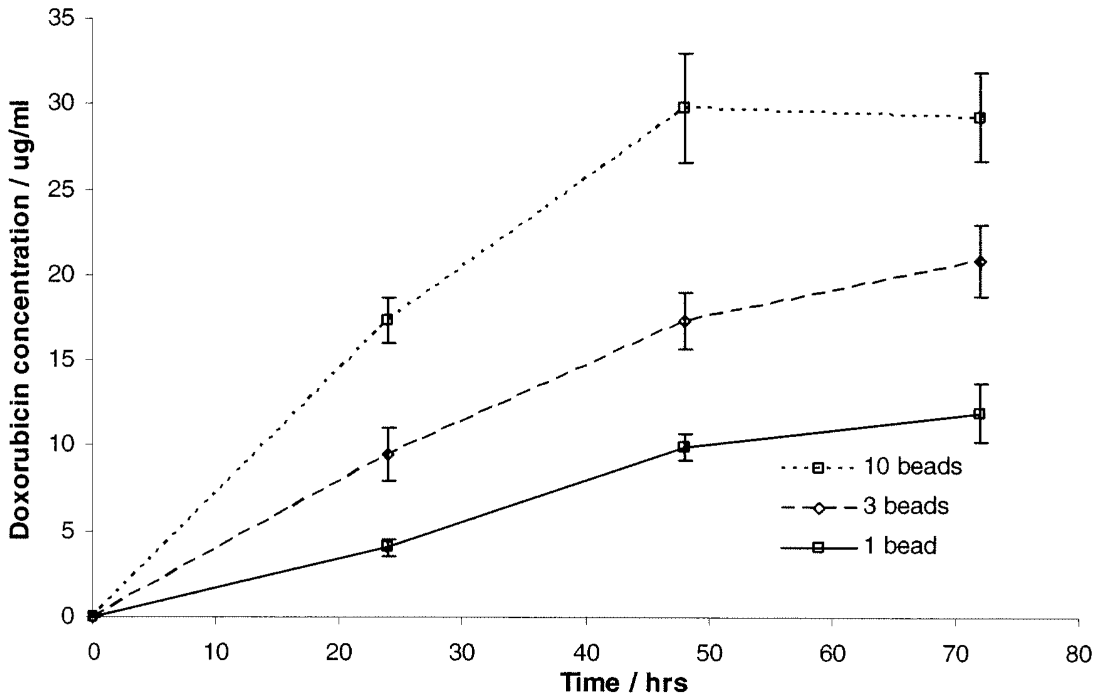


Figure 13

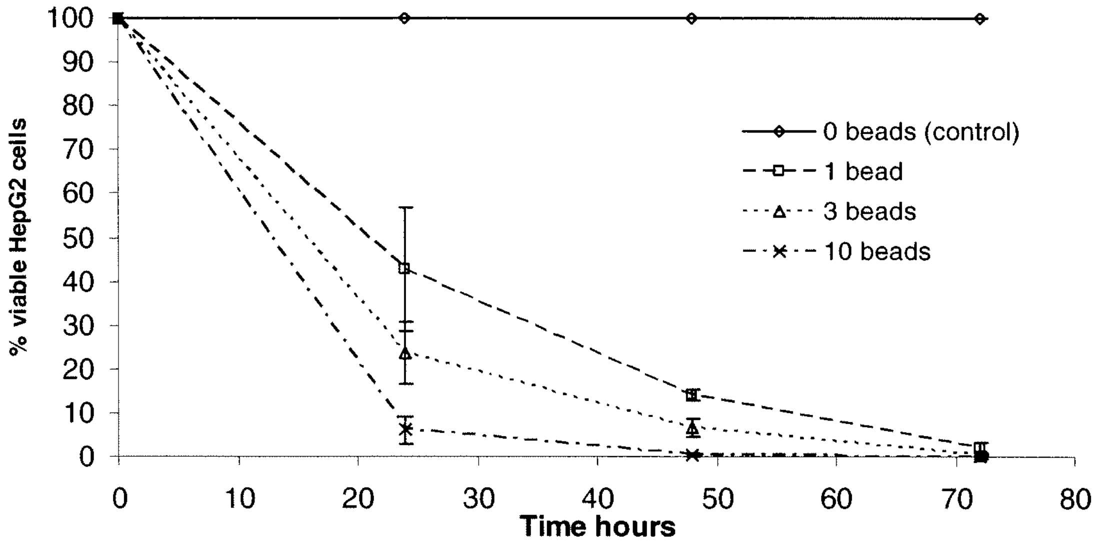


**Figure 14**

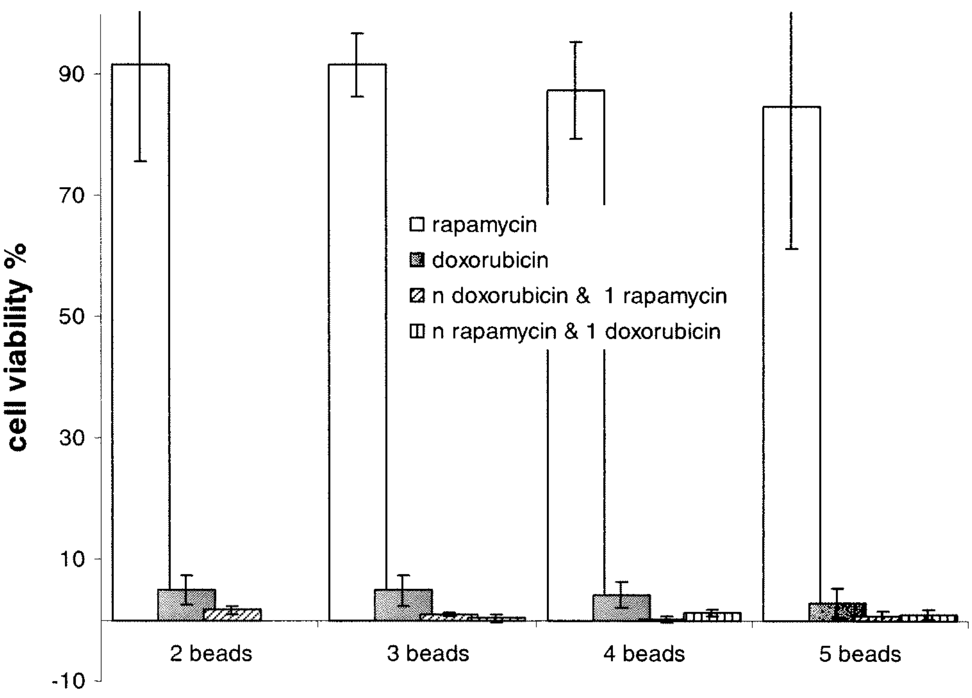


**Figure 15**

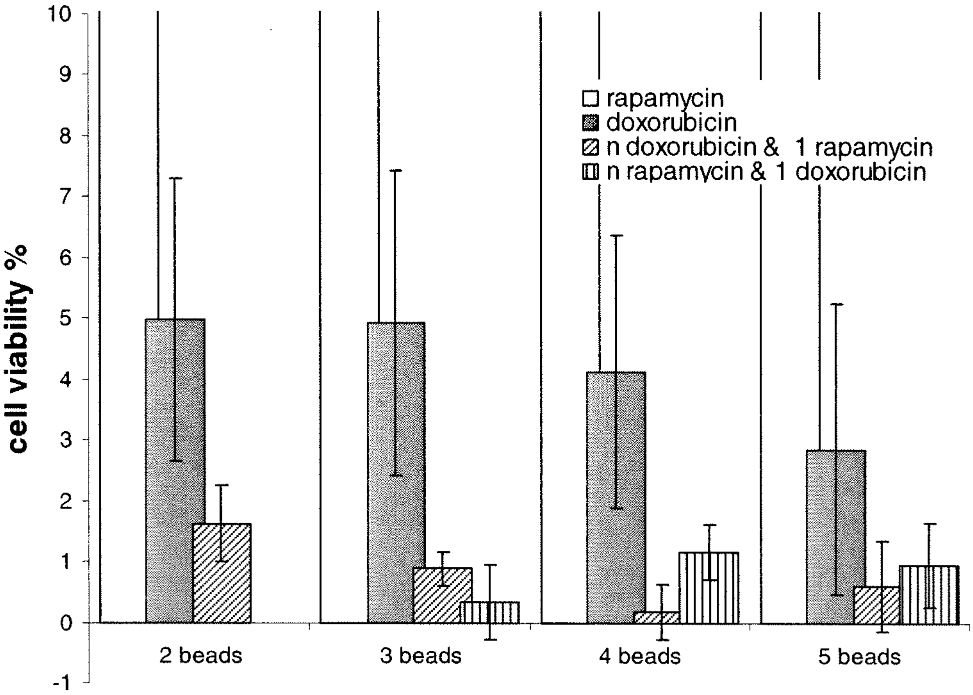


**Figure 16**

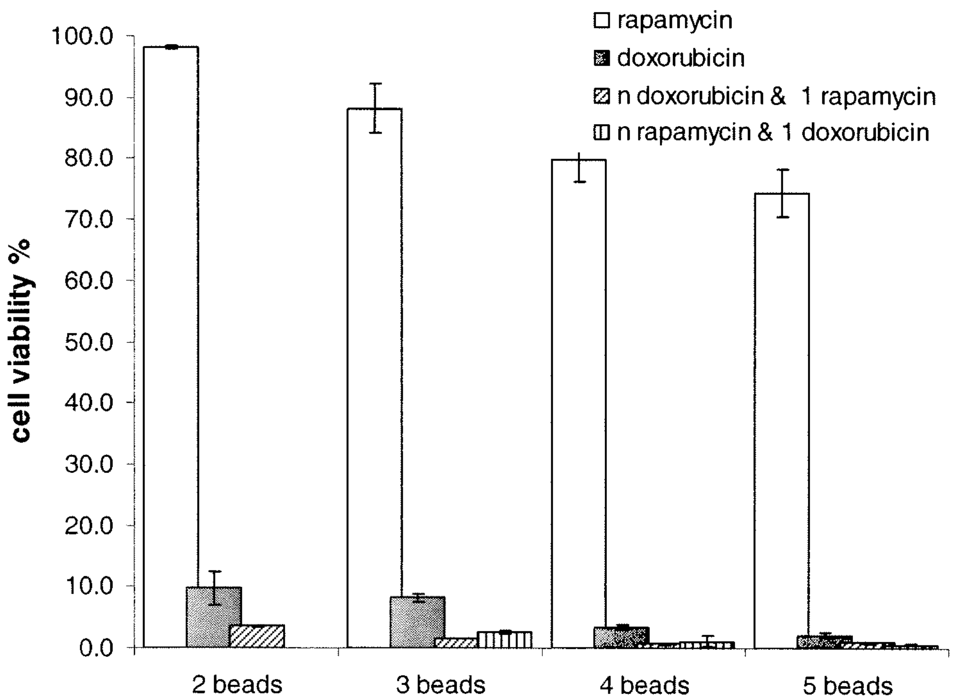


**Figure 17**

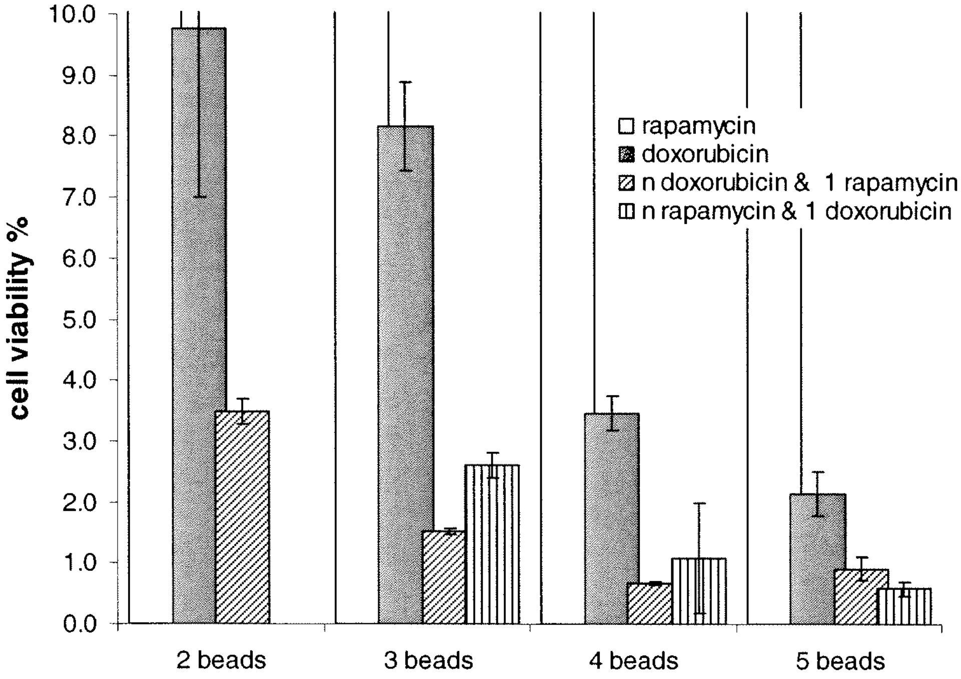


**Figure 18**

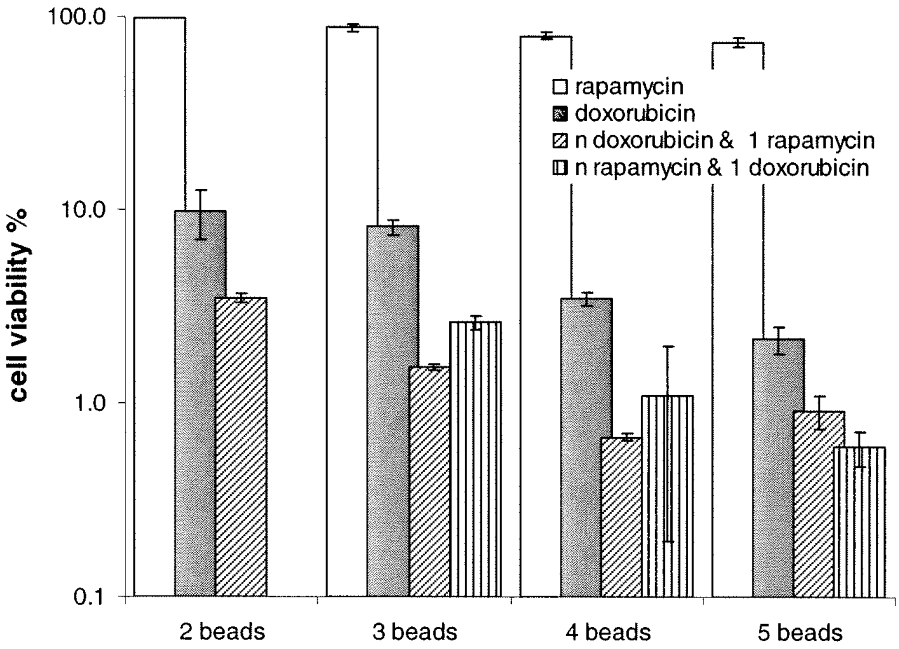


**Figure 19**

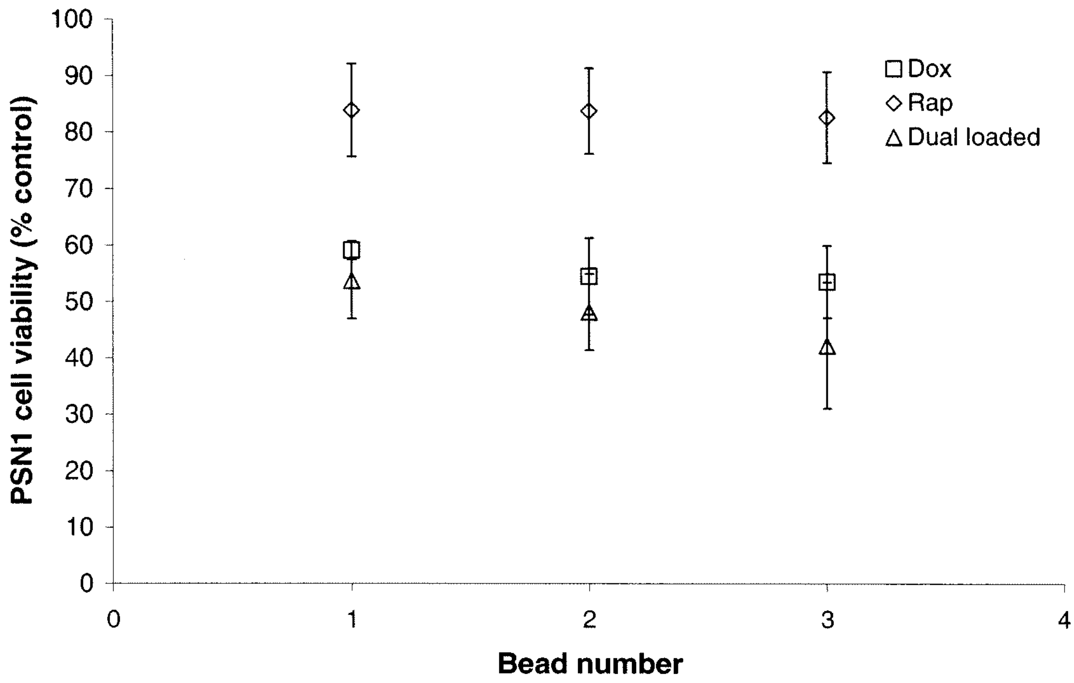


Figure 20

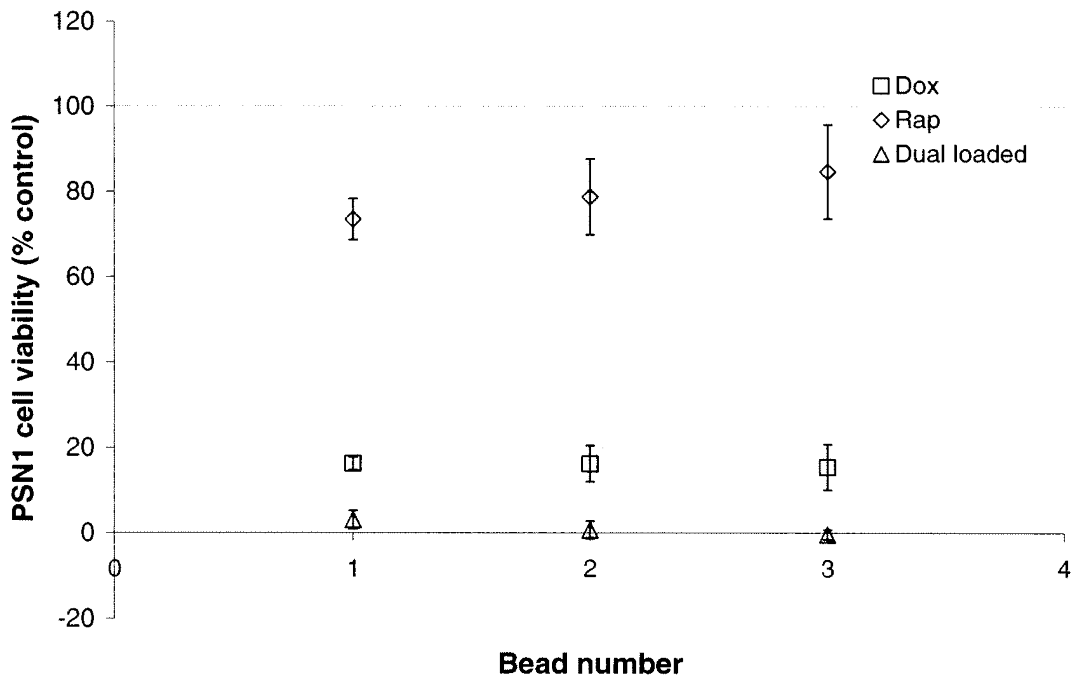


Figure 21

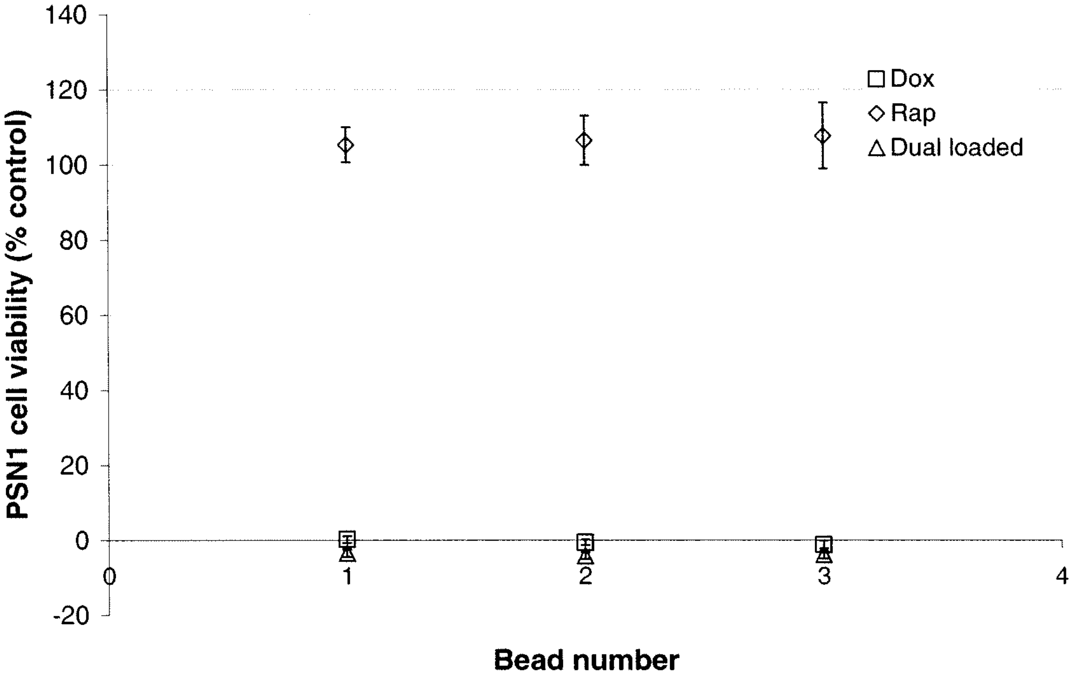


Figure 22

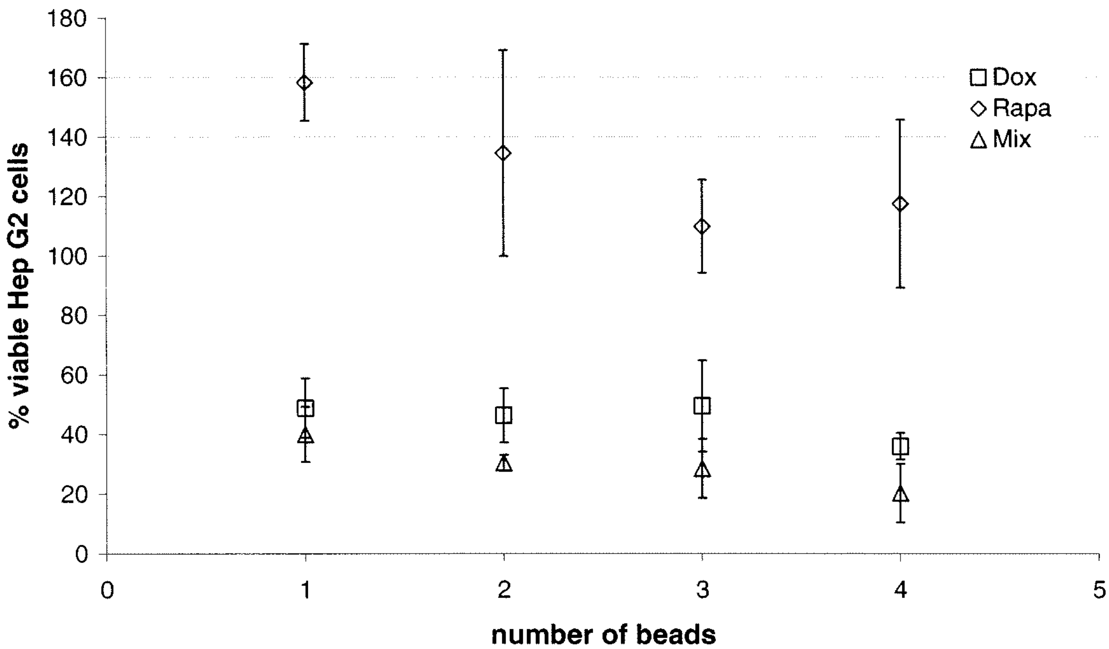


Figure 23

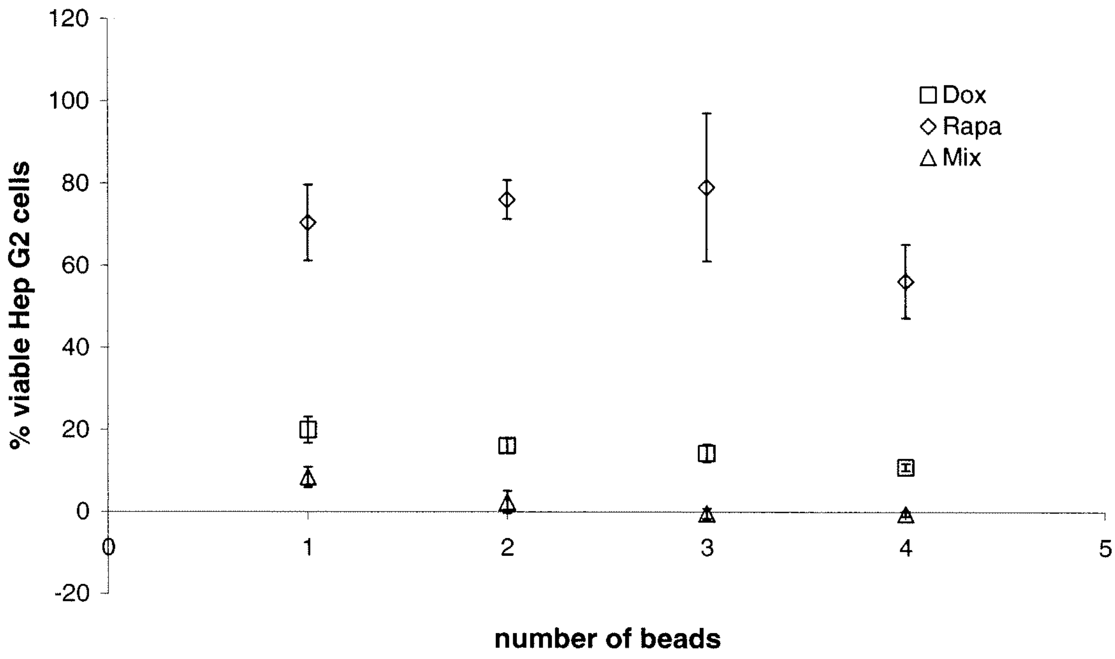


Figure 24

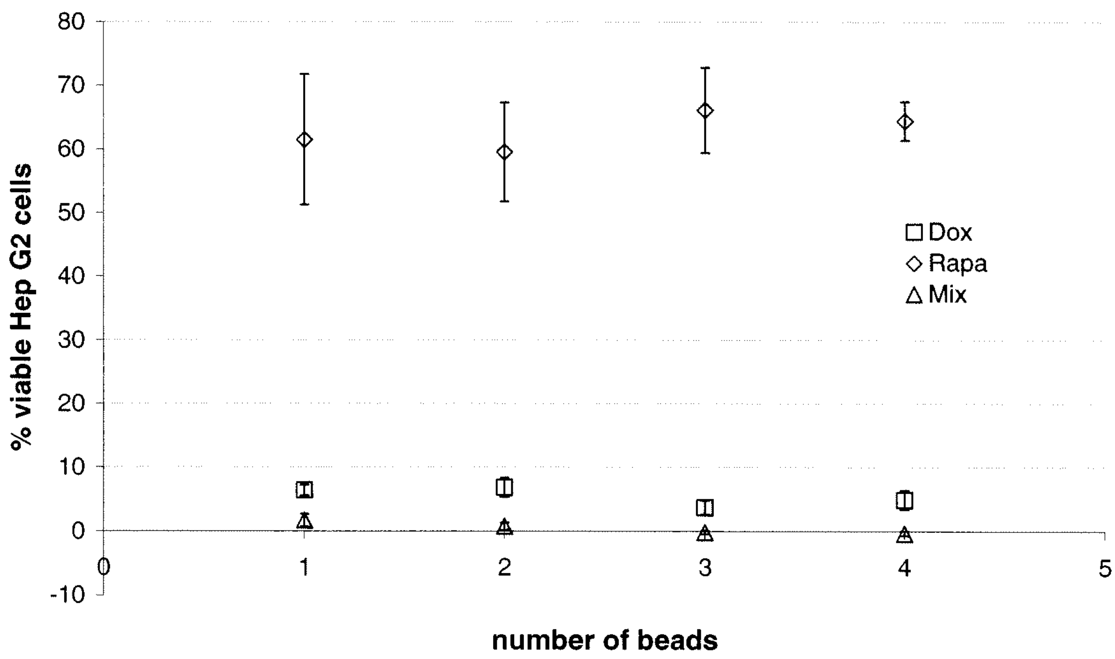


Figure 25

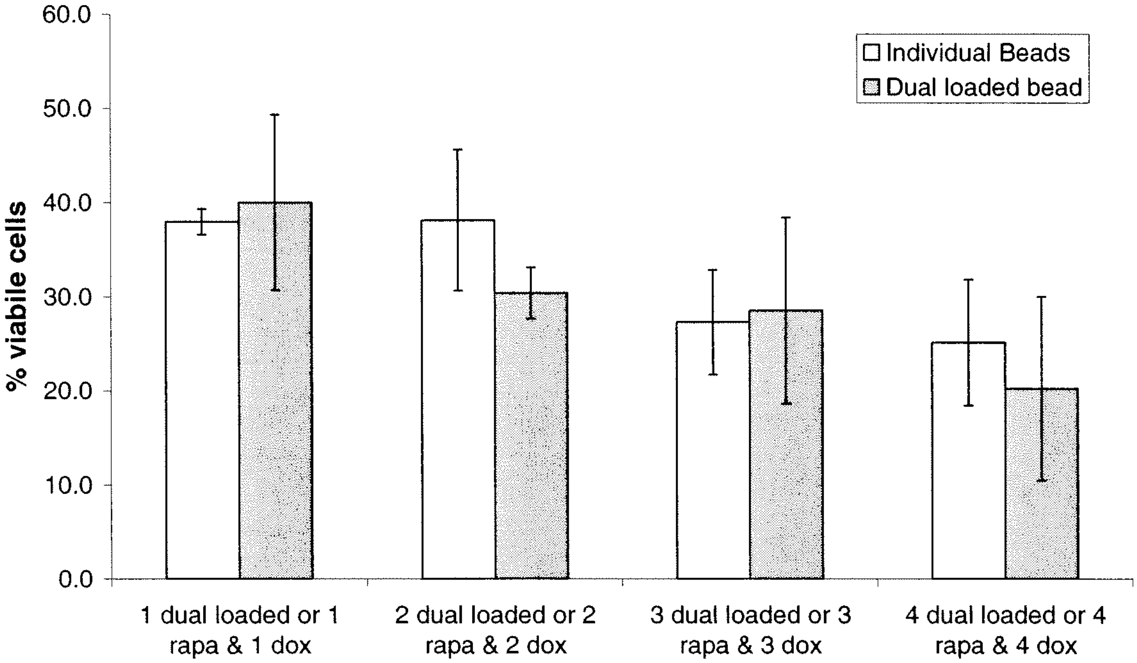


Figure 26

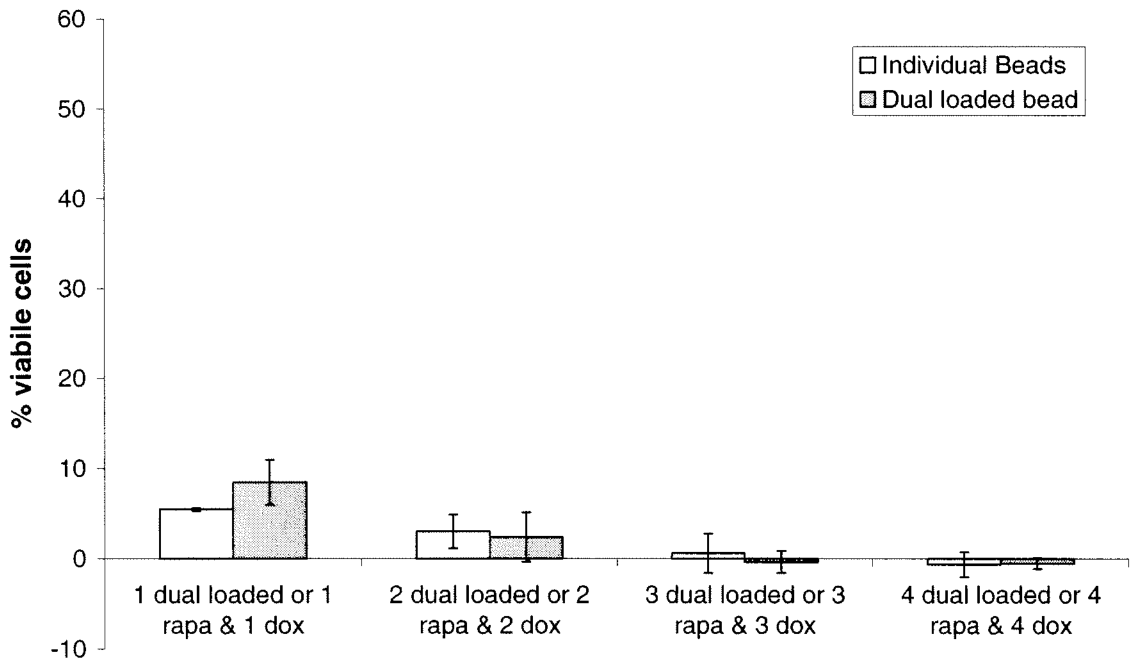


Figure 27

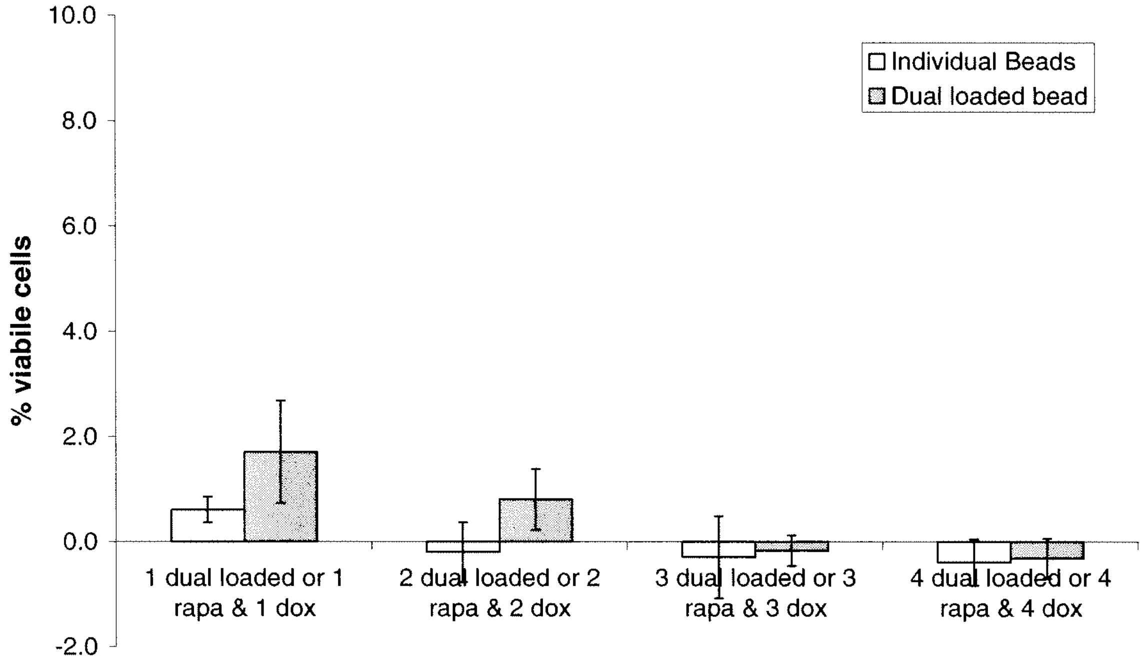
% viabile cells
10.0
8.0
6.0
4.0
2.0
0.0
-2.0
Individual Beads
Dual loaded bead
1 dual loaded or 1 rapa & 1 dox
2 dual loaded or 2 rapa & 2 dox
3 dual loaded or 3 rapa & 3 dox
4 dual loaded or 4 rapa & 4 dox

Figure 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003028696 A
- WO 2004087115 A
- WO 2004087105 A
- WO 2004093795 A
- WO 2006055903 A
- EP 551162 A
- EP 0568130 A
- WO 2003022807 A
- WO 2004022124 A
- WO 9850018 A
- WO 2004071495 A
- WO 2004073688 A
- WO 2007085615 A
- WO 2006013376 A
- WO 2007090897 A
- WO 2006027567 A
- WO 04071495 A
- EP 0321122 A
- US 4978713 A
- US 5508317 A
- US 5583163 A
- WO 0029481 A
- WO 9207885 A
- WO 9416748 A
- WO 9416749 A
- WO 9520407 A
- US 4224427 A
- WO 0152915 A
- EP 200750690 W


### Non-patent literature cited in the description


- **BECERRA.** *Oncology,* 2004, vol. 18, 46-48
- **NISHINO et al.** *Gynecol Oncol,* 2005, vol. 97, 893-897
- **BAEK et al.** *Br J Cancer,* 2006, vol. 25, 312-320
- **CHOU.** *J. Biol. Chem.,* 1977, vol. 252 (18), 6438-6442
- **CHOU-TALALAY.** *T.I.P.S.,* November 1983, 450-454
- **TARDI, P. G. et al.** *Biochim. Biophys. Acta,* 2007, vol. 1768, 678-587
- **SHARMA R et al.** *Pharm Research,* 2001, vol. 18, 1405-1410
- **GUPTE A ; CIFTCI K.** *Int. J. Pharm,* 2004, vol. 276 (1-2), 93-106
- **CHANDY et al.** Development of Poly(Lactic Acid)/Chitosan Co-Matrix Microspheres: Controlled Release of Taxol-Heparin for Preventing Restenosis. *Drug delivery,* 2001, vol. 8, 77-86
- **LAMPRECHT et al.** *Eur J Pharm and Biopharm,* 2005, vol. 59, 367-371
- **LIU et al.** *J. Pharm. Pharmacol.,* 2003, vol. 55, 1063-1073
- **LIU et al.** *J. Pharm. Sci,* 2000, vol. 8-9 (6), 807-817
- **VARELA et al.** *J Hepatology,* 2007, vol. 46, 474-81
- **BOROVAC T et al.** *J Control Release,* 2006, vol. 115, 266-74
- **SAWAOKA et al.** *J Clin Gastroenterol,* 1998, vol. 27, 47-52
- **SHEN et al.** *Cancer Research,* 1998, vol. 58, 362-366
- **YAMAUCHI et al.** *Anticancer Res,* 2003, vol. 23, 245-249
- **YAO et al.** *Br J Cancer,* 2004, vol. 90, 712-719
- **CUI et al.** *Clin Cancer Res,* 2005, vol. 11, 8213-8221
- **MURATA et al.** *Dig Dis Sci,* 2005, vol. 50, 70-75
- **CHOU J.** *Th. Biol.,* vol. 59, 253-276
- **WENDEL,H-G et al.** *Nature,* 2004, vol. 428, 332-337
- **AVELLINO,R. et al.** *Blood,* 2005, vol. 106 (4), 1400-1406
- **MONDESIRE WH et al.** *Clinical Cancer Research,* 2004, vol. 10, 7031-7042
- **BEUVINK I et al.** *Cell,* 2005, vol. 120, 747-759
- **TOFFOLI G et al.** *Biochem Pharmacol,* 1995, vol. 50, 1245-55
- **PAULI-MAGNUS C et al.** *J Pharmacol Exp Ther,* 2000, vol. 293, 376-82